(19) Europäisches Patentamt / European Patent Office / Office européen des brevets

(11) EP 2 358 343 B1

(12) EUROPÄISCHE PATENTSCHRIFT

(45) Veröffentlichungstag und Bekanntmachung des Hinweises auf die Patenterteilung:
**19.10.2016 Patentblatt 2016/42**

(21) Anmeldenummer: **09760875.6**

(22) Anmeldetag: **30.11.2009**

(51) Int Cl.:
***A61Q 5/00*** *(2006.01)* ***A61Q 5/10*** *(2006.01)*
***A61K 8/40*** *(2006.01)* ***A61K 8/49*** *(2006.01)*

(86) Internationale Anmeldenummer:
**PCT/EP2009/066044**

(87) Internationale Veröffentlichungsnummer:
**WO 2010/072512 (01.07.2010 Gazette 2010/26)**

# (54) VERFAHREN ZUM ENTFÄRBEN KERATINHALTIGER FASERN

METHOD FOR DECOLOURING KERATINOUS FIBRES

PROCÉDÉ DE DÉCOLORATION DE FIBRES KÉRATINIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **16.12.2008 DE 102008062239**

(43) Veröffentlichungstag der Anmeldung:
**24.08.2011 Patentblatt 2011/34**

(73) Patentinhaber: **Henkel AG & Co. KGaA**
**40589 Düsseldorf (DE)**

(72) Erfinder:
- **OBERKOBUSCH, Doris**
  **40591 Düsseldorf (DE)**
- **GROß, Wibke**
  **41836 Hückelhoven (DE)**
- **STEPHAN, Laura**
  **40589 Düsseldorf (DE)**

(56) Entgegenhaltungen:
**EP-A2- 2 014 275** **WO-A1-01/85111**
**WO-A1-2004/022016** **WO-A1-2005/051336**
**WO-A2-2004/058203** **WO-A2-2005/120445**
**WO-A2-2010/046256** **DE-A1-102007 047 685**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 358 343 B1

Printed by Jouve, 75001 PARIS (FR)

**Beschreibung**

Die vorliegende Erfindung betrifft die ein Verfahren zur Entfärbung gefärbter keratinhaltiger Fasern, die mit einem anwendungsbereiten Färbemittel, enthaltend in einem kosmetischen Träger eine Kombination aus mindestens einer CH-aciden Verbindung mit mindestens einer reaktiven Carbonylverbindung, gefärbt wurden, wobei das Entfärbemittel Wasserstoffperoxid enthält und frei von organischen Peroxoverbindungen und von anorganischen Persalzen ist.

Zum Färben von Haaren sind dem Fachmann verschiedene Färbesysteme bekannt, welche sich in der Haltbarkeit der Färbung auf dem Haar und dem Schädigungsgrad, welchen das Haar während des Haarfärbeprozesses erleidet, stark unterscheiden.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraamino-pyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 4-Amino-3-methylphenol, 2-Aminomethyl-4-aminophenol, 4,5-Diamino-1-(2-hydroxyethyl)pyrazol, 2-Hydroxy-4,5,6-triaminopyrimidin, 2,4-Dihydroxy-5,6-diaminopyrimidin, 2,5,6-Triamino-4-hydroxypyrimidin und N,N'-Bis(2-hydroxyethyl)-N,N'-bis(4-aminophenyl)-1,3-diaminopropan-2-ol.

Als Kupplerkomponenten werden in der Regel m-Phenylendiaminderivate, Naphthole, Pyridinderivate, Resorcin und Resorcinderivate, Pyrazolone und m-Aminophenole verwendet. Als Kupplersubstanzen eignen sich insbesondere 1-Naphthol, 1,5-Dihydroxynaphthalin, 2,7-Dihydroxynaphthalin, 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazol-5-on, 2,4-Dichlor-3-aminophenol, 1,3-Bis-(2,4-diaminophenoxy)-propan, 2-Amino-3-hydroxypyridin, 4-Chlorresorcin, 2-Chlor-6-methyl-3-aminophenol, 2-Methylresorcin, 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Eine weitere Möglichkeit zur Farbveränderung bietet die Verwendung von Färbemitteln, welche sogenannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen sind nahezu permanent und besitzen überwiegend Farbechtheiten auf der keratinhaltigen Faser, die mit denen der Oxidationsfärbung vergleichbar sind.

Das mit der schonenden Oxofärbung erzielbare Nuancenspektrum ist sehr breit, und die erhaltene Färbung weist oftmals eine akzeptable Brillanz und Farbtiefe auf. Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Da bei der Oxofärbung auch ohne den Einsatz von Oxidationsmitteln wie beispielsweise Wasserstoffperoxid leuchtende Farben mit guter Haltbarkeit auf dem Haar erzielt werden können, ist diese Färbemethode mit einer geringen Haarschädigung verbunden und vor diesem Hintergrund für den Verbraucher von besonderem Interesse.

Die technische Realisierung der Oxofärbung wird beispielsweise in WO 2004/022016 A1 und WO 2005/120445 A2 offenbart.

In DE 10022743 A1 wird ein Mittel zum Färben von Haaren beschrieben, welches durch Vermischen einer ein Enamin oder dessen Säureadditionssalz enthaltenden Komponente (A1) und einer eine Carbonylverbindung und ein primäres Amin enthaltenden Komponente (A2) hergestellt wird. Hierbei weist die Komponente A1 einen sauren pH-Wert auf, die Komponente A2 besitzt jedoch einen alkalischen pH-Wert.

Ein wichtiges technisches Gebiet stellt neben dem Färben das Abziehen von Farbstoffen dar. Darunter versteht man im Allgemeinen das Entfernen von Färbungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Zur Erreichung eines erfolgreichen Farbabzuges permanenter Oxidationsfärbungen reicht der Einsatz von Wasserstoffperoxid als Oxidationsmittel allein nicht aus. Der Fachmann setzt bekanntermaßen zum Farbabzug permanenter Oxidationsfärbungen kosmetische Entfärbemittel ein, die neben Wasserstoffperoxid zusätzlich entweder mindestens eine organische Peroxoverbindung (wie beispielsweise organische Persäuren wie Perphthalsäure, Peressigsäure), oder mindestens ein anorganisches Persalz (wie beispielsweise Salze der Persulfate, Perborate oder Peroxodisulfate) enthalten. Diese Entfärbemittel entsprechen beispielsweise den bekannten Blondiermitteln zur Ultrablondierung von Haaren. Nach der Anwendung der stark oxidierend wirkenden Ultrablondiermittel können die zuvor beschriebenen physikalischen Änderungen des Substrats in unterschiedlich starker Ausprägung beobachtet werden.

Aufgabe der vorliegenden Erfindung ist es daher, im Rahmen der Oxofärbung ein Verfahren zum Färben und Entfärben keratinhaltiger Fasern zur Verfügung zu stellen, das den Verbraucher in die Lage versetzt, neben der permanenten, faserschonenden Oxofärbung einen möglichst vollständigen, faserschonenden Farbabzug durchzuführen. Die keratinhaltige Faser soll nach dem Farbabzug möglichst vollständig von ihrer künstlich aufgebrachten Oxofärbung befreit sein, keine oder eine möglichst geringe Schädigung der Faserstruktur erhalten und insbesondere einen weichen Griff sowie eine gute Kämmbarkeit aufweisen.

Überraschenderweise konnte nun gefunden werden, dass wasserstoffhaltige Entfärbemittel einen hervorragenden Farbabzug der mit der Oxofärbung gefärbten keratinhaltigen Fasern bewirken und gleichzeitig die Fasern geschont werden. Dabei kann auf den Einsatz von organischen Peroxoverbindungen und anorganischen Persalzen verzichtet werden. Außerdem glückt die faserschonende Entfärbung der permanenten Oxofärbung bereits unter Einsatz geringer Mengen Wasserstoffperoxid.

Ein erster Gegenstand der Erfindung ist daher ein Verfahren zum Entfärben gefärbter keratinhaltiger Fasern, insbesondere menschlicher Haare, dadurch gekennzeichnet dass

- auf die mit einem Färbemittel, enthaltend in einem kosmetischen Träger eine Kombination aus mindestens einer CH-aciden Verbindung, die aus mindestens einer Verbindung der Formel (CH-1) und/oder aus mindestens einer Verbindung der Formel (CH-2) ausgewählt wird,

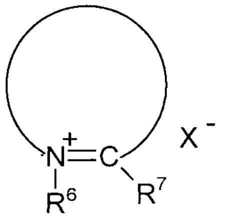

(CH-1)

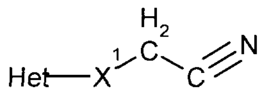

(CH-2)

worin

- $R^6$ steht für eine lineare oder cyclische ($C_1$ bis $C_6$)-Alkylgruppe, eine ($C_2$ bis $C_6$)-Alkenylgruppe, eine gegebenenfalls substituierte Arylgruppe, eine gegebenenfalls substituierte Heteroarylgruppe, eine Aryl-($C_1$ bis $C_6$)-alkylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxy-($C_1$ bis $C_6$)-alkylgruppe, eine Gruppe $R^IR^{II}N$-$(CH_2)_m$- worin $R^I$ und $R^{II}$ stehen unabhängig voneinander für ein Wasserstoffatom, eine ($C_1$ bis $C_4$)-Alkylgruppe, eine ($C_1$ bis $C_4$)-Hydroxyalkylgruppe oder eine Aryl-($C_1$ bis $C_6$)-alkylgruppe, wobei $R^I$ und $R^{II}$ gemeinsam mit dem Stickstoffatom einen 5-, 6- oder 7-gliedrigen Ring bilden können und m steht für eine Zahl 2, 3, 4, 5 oder 6,
- $R^7$ steht für eine ($C_1$ bis $C_6$)-Alkylgruppe, insbesondere für eine Methylgruppe,

- $X^-$ steht für ein physiologisch verträgliches Anion,
- der Zyklus der Formel (CH-1) repräsentiert alle Ringsstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
- Het steht für einen gegebenenfalls substituierten Heteroaromaten,
- $X^1$ steht für eine direkte Bindung oder eine Carbonylgruppe, mit mindestens einer reaktiven Carbonylverbindung, gefärbten keratinhaltigen Fasern ein Entfärbemittel, enthaltend in einem kosmetischen Träger Wasserstoffperoxid, aufgebracht, 1 bis 30 Minuten auf der Faser belassen und anschießend wieder ausgespült wird, und

- das Entfärbemittel frei von organischen Peroxoverbindungen und von anorganischen Persalzen ist.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierte Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurtehan und Polyesterfasern verwendet werden.
Getrennt konfektioniert bedeutet im Sinne der Erfindung, dass die betroffenen Mittel jeweils in einem eigenen Container konfektioniert sind. Als Container können unterschiedliche Verpackungsarten dienen, wie beispielsweise Folien, Beutel, Flaschen, Dosen, Aerosolbehältnisse. Ebenso gilt eine Kammer eines Mehrkammerbehältnisses als Container.
Entfärben bedeutet im Sinne der Erfindung den Farbabzug der durch die anwendungsbereiten Färbemittel des Kits erzielten Färbungen unter weitgehender Erhaltung der natürlichen Farbe der keratinhaltigen Faser bzw. der natürlichen Haarfarbe.
Das erfindungsgemäße Verfahren stellt ein effektives und faserschonendes Färbe- und Entfärbesystem dar. Folglich ist es im Rahmen des zuvor beschriebenen Verfahrens erfindungsgemäß vorgesehen, dass

- zur Färbung der keratinhaltiger Fasern mindestens ein Mittel A1 und mindestens ein Mittel A2 zu einem anwendungsbereiten Färbemittel vermischt werden und
- die mit dem anwendungsbereiten Färbemittel gefärbten keratinhaltigen Fasern bei Bedarf mit dem Entfärbemittel B entfärbt werden.

In einer bevorzugten Ausführungsform des erfindungsgemäßen Verfahrens sind die CH-aciden Verbindungen und die reaktiven Carbonylverbindungen des anwendungsbereiten Färbemittels getrennt voneinander konfektioniert und liegen in mindestens einem Mittel A1 und mindestens einem Mittel A2 vor, mit den Maßgaben, dass

- das Mittel A1 in einem kosmetischen Träger mindestens eine CH-acide Verbindung enthält und
- das Mittel A2 in einem kosmetischen Träger mindestens eine reaktive Carbonylverbindung enthält. Im Rahmen dieser Ausführungsform sind die Mittel A1 somit frei von reaktiven Carbonylverbindungen und die Mittel A2 frei von CH-aciden Verbindungen.

Das anwendungsbereite Färbemittel - beziehungsweise das Mittel A1 - enthält mindestens eine CH-acide Verbindung.
Als CH-acide Verbindungen werden solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-WasserstoffBindung bewirkt wird. Besagtes Wasserstoffatom kann mit Hilfe einer Base abstrahiert werden.
Das anwendungsbereite Färbemittel enthält mindestens eine CH-acide Verbindung ausgewählt aus mindestens einer Verbindung der Formel (CH-1) und/oder aus mindestens einer Verbindung der Formel (CH-2),

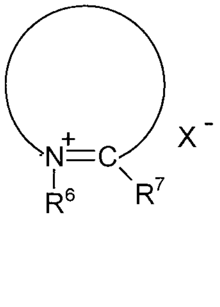
(CH-1)

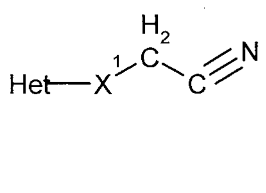
(CH-2)

worin

- $R^6$ steht für eine lineare oder cyclische ($C_1$ bis $C_6$)-Alkylgruppe, eine ($C_2$ bis $C_6$)-Alkenylgruppe, eine gegebenenfalls substituierte Arylgruppe, eine gegebenenfalls substituierte Heteroarylgruppe, eine Aryl-($C_1$ bis $C_6$)-alkylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxy-($C_1$ bis $C_6$)-alkylgruppe, eine Gruppe $R^IR^{II}N-(Ch_2)_m-$, worin $R^I$ und $R^{II}$ stehen unabhängig voneinander für ein Wasserstoffatom, eine ($C_1$ bis $C_4$)-Alkylgruppe, eine ($C_1$ bis $C_4$)-Hydroxyalkylgruppe oder eine Aryl-($C_1$ bis $C_6$)-alkylgruppe, wobei $R^I$ und $R^{II}$ gemeinsam mit dem Stickstoffatom einen 5-, 6- oder 7-gliedrigen Ring bilden können und m steht für eine Zahl 2, 3, 4, 5 oder 6,
- $R^7$ steht für eine ($C_1$ bis $C_6$)-Alkylgruppe, insbesondere für eine Methylgruppe,
- $X^-$ steht für ein physiologisch verträgliches Anion,
- der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
- Het steht für einen gegebenenfalls substituierten Heteroaromaten,
- $X^1$ steht für eine direkte Bindung oder eine Carbonylgruppe.

Die Verbindungen gemäß Formel (CH-1) und (CH-2) sind CH-acide Verbindungen. Aus den kationischen Verbindungen der Formel (CH-1) läßt sich durch Zugabe einer Base, welche die Abspaltung eines Protons bewirkt, gezielt die entsprechende ungeladene Enaminform darstellen. Exemplarisch für Verbindungen gemäß Formel (CH-1) wird die Darstellung der Enaminform nachfolgend anhand der Formeln (CH-1-A) und (CH-1-B) mit $R^7 = CH_3$ illustriert. Auch die entsprechenden Enaminformen der CH-aciden Verbindungen gemäß Formel (CH-1) sind erfindungsgemäß.

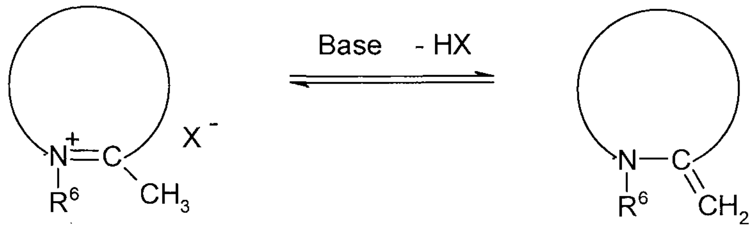


(CH-1-A) (CH-1-B)

Es ist erfindungsgemäß besonders bevorzugt, die Verbindungen der Formel (CH-1) auszuwählen aus mindestens einer Verbindung der Formel (CH-3),

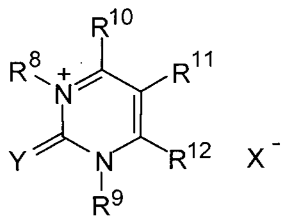

(CH-3)

worin

- $R^8$ und $R^9$ stehen unabhängig voneinander für eine lineare oder cyclische ($C_1$ bis $C_6$)-Alkylgruppe, eine ($C_2$ bis $C_6$)-Alkenylgruppe, eine gegebenenfalls substituierte Arylgruppe, eine gegebenenfalls substituierte Heteroarylgruppe, eine Aryl-($C_1$ bis $C_6$)-alkylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxy-($C_1$ bis $C_6$)-alkylgruppe, eine Gruppe $R^IR^{II}N-(CH_2)_m-$, worin $R^I$ und $R^{II}$ stehen unabhängig voneinander für ein Wasserstoffatom, eine ($C_1$ bis $C_4$)-Alkylgruppe, eine ($C_1$ bis $C_4$)-Hydroxyalkylgruppe oder eine Aryl-($C_1$ bis $C_4$)-alkylgruppe, wobei $R^I$ und $R^{II}$ gemeinsam mit dem Stickstoffatom einen 5-, 6- oder 7-gliedrigen Ring bilden können und m steht für eine Zahl 2, 3, 4, 5 oder 6,
- $R^{10}$ und $R^{12}$ stehen unabhängig voneinander für ein Wasserstoffatom oder eine $C_1$-$C_6$-Alkylgruppe, wobei mindestens einer der Reste $R^{10}$ und $R^{12}$ eine ($C_1$ bis $C_6$)-Alkylgruppe bedeutet,
- $R^{11}$ steht für ein Wasserstoffatom, eine ($C_1$ bis $C_6$)-Alkylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxygruppe, eine ($C_1$ bis $C_6$)-Hydroxyalkoxygruppe, eine Gruppe

$R^{III}R^{IV}N-(CH_2)_q-$, worin $R^{III}$ und $R^{IV}$ stehen unabhängig voneinander für ein Wasserstoffatom, eine ($C_1$ bis $C_6$)-Alkylgruppe, eine ($C_1$ bis $C_6$)-Hydroxyalkylgruppe oder eine Aryl-($C_1$ bis $C_6$)-alkylgruppe und q steht für eine Zahl 1, 2, 3, 4, 5 oder 6, wobei der Rest $R^{11}$ zusammen mit einem der Reste $R^{10}$ oder $R^{12}$ einen 5- oder 6-gliedrigen aromatischen Ring bilden kann, der gegebenenfalls mit einem Halogenatom, einer ($C_1$ bis $C_6$)-Alkylgruppe, einer ($C_1$ bis $C_6$)-Hydroxyalkylgruppe, einer ($C_2$ bis $C_6$)-Polyhydroxyalkylgruppe, einer ($C_1$ bis $C_6$)-Alkoxygruppe, einer ($C_1$ bis $C_6$)-Hydroxyalkoxygruppe, einer Nitrogruppe, einer Hydroxygruppe, einer Gruppe $R^VR^{VI}N-(CH_2)_s-$, worin $R^V$ und $R^{VI}$ stehen unabhängig voneinander für ein Wasserstoffatom, eine ($C_1$ bis $C_6$)-Alkylgruppe, eine ($C_1$ bis $C_6$)-Hydroxyalkylgruppe oder eine Aryl-($C_1$ bis $C_6$)-alkylgruppe und s steht für eine Zahl 0, 1, 2, 3, 4, 5 oder 6 substituiert sein kann,

- Y steht für ein Sauerstoffatom, ein Schwefelatom oder eine Gruppe $NR^{VII}$, worin $R^{VII}$ steht für ein Wasserstoffatom, eine Arylgruppe, eine Heteroarylgruppe, eine ($C_1$ bis $C_6$)-Alkylgruppe oder eine Aryl-($C_1$ bis $C_6$)-alkylgruppe,
- $X^-$ steht für ein physiologisch verträgliches Anion.

Mindestens eine Gruppe $R^{10}$ oder $R^{12}$ gemäß Formel (CH-3) steht zwingend für eine ($C_1$ bis $C_6$)-Alkylgruppe. Diese Alkylgruppe trägt an deren $\alpha$-Kohlenstoffatom bevorzugt mindestens zwei Wasserstoffatome. Besonders bevorzugte Alkylgruppen sind die Methyl-, Ethyl-, Propyl-, n-Butyl-, isoButyl, n-Pentyl-, neo-Pentyl-, n-Hexylgruppe. Ganz besonders bevorzugt stehen $R^{10}$ und $R^{12}$ unabhängig voneinander für Wasserstoff oder eine Methylgruppe, wobei mindestens eine Gruppe $R^{10}$ oder $R^{12}$ eine Methylgruppe bedeutet.

In einer bevorzugten Ausführungsform steht Y der Formel (CH-3) für ein Sauerstoff- oder ein Schwefelatom, besonders bevorzugt für ein Sauerstoffatom.

Der Rest $R^8$ der Formel (CH-3) wird bevorzugt ausgewählt aus einer ($C_1$ bis $C_6$)-Alkylgruppe (besonders bevorzugt einer Methylgruppe), einer ($C_2$ bis $C_6$)-Alkenylgruppe (insbesondere einer Allylgruppe), einer ($C_2$ bis $C_6$)-Hydroxyalkylgruppe (insbesondere eine 2-Hydroxyethylgruppe) oder einer gegebenenfalls substituierten Benzylgruppe.

$R^{11}$ der Formel (CH-3) steht bevorzugt für ein Wasserstoffatom.

Besonders bevorzugt stehen in Formel (CH-3) die Reste $R^9$, $R^{10}$ und $R^{12}$ für eine Methylgruppe, der Rest $R^{11}$ für ein Wasserstoffatom, Y für ein Sauerstoff- oder ein Schwefelatom und der Rest $R^8$ wird ausgewählt aus einer ($C_1$ bis $C_6$)-Alkylgruppe (besonders bevorzugt einer Methylgruppe), einer ($C_2$ bis $C_6$)-Alkenylgruppe (insbesondere einer Allylgruppe), einer ($C_2$ bis $C_6$)-Hydroxyalkylgruppe (insbesondere eine 2-Hydroxyethylgruppe) oder einer gegebenenfalls substituierten Benzylgruppe.

Ganz besonders brillante Haarfärbungen können erhalten werden, wenn die in anwendungsbereite Färbemittel enthaltene C,H-acide Verbindung der Formel (CH-1) und/oder der Formel (CH-2) eine Verbindung ausgewählt aus mindestens einer Verbindung der Gruppe bestehend aus 2-(2-Furoyl)-acetonitril, 2-(5-Brom-2-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1H-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 2-(6-Hydroxy-4,7-dimethoxy-1-benzofuran-5-oyl)-acetonitril und den Salzen mit physiologisch verträglichem Gegenion X- des 1,2-Dihydro-1,3,4,6-tetramethyl-2-oxo-pyrimidiniums, 1,2-Dihydro-1,3-diethyl-4,6-dimethyl-2-oxo-pyrimidiniums, 1,2-Dihydro-1,3-dipropyl-4,6-dimethyl-2-oxo-pyrimidiniums, 1,2-Dihydro-1,3-di(2-hydroxyethyl)-4,6-dimethyl-2-oxo-pyrimidiniums, 1,2-Dihydro-1,3-diphenyl-4,6-dimethyl-2-oxo-pyrimidiniums, 1,2-Dihydro-1,3,4-trimethyl-2-oxo-pyrimidiniums, 1,2-Dihydro-1,3-diethyl-4-methyl-2-oxo-pyrimidiniums, 1,2-Dihydro-1,3-dipropyl-4-methyl-2-oxo-pyrimidiniums, 1,2-Dihydro-1,3-di(2-hydroxyethyl)-4-methyl-2-oxo-pyrimidiniums, 1,2-Dihydro-1,3-diphenyl-4-methyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums, 1,2-Dihydro-1,3,4,6-tetramethyl-2-thioxo-pyrimidiniums, 1,2-Dihydro-1,3-diethyl-4,6-dimethyl-2-thioxo-pyrimidiniums, 1,2-Dihydro-1,3-dipropyl-4,6-dimethyl-2-thioxo-pyrimidiniums, 1,2-Dihydro-1,3-di(2-hydroxyethyl)-4,6-dimethyl-2-thioxo-pyrimidiniums, 1,2-Dihydro-1,3-diphenyl-4,6-dimethyl-2-thioxo-pyrimidiniums, 1,2-Dihydro-1,3,4-trimethyl-2-thioxo-pyrimidiniums, 1,2-Dihydro-1,3-diethyl-4-methyl-2-thioxo-pyrimidiniums, 1,2-Dihydro-1,3-dipropyl-4-methyl-2-thioxo-pyrimidiniums, 1,2-Dihydro-1,3-di(2-hydroxyethyl)-4-methyl-2-thioxo-pyrimidiniums, 1,2-Dihydro-1,3-diphenyl-4-methyl-2-thioxo-pyrimidiniums, 1,2-Dihydro-3,4-dimethyl-2-oxo-chinazoliniums und 1,2-Dihydro-3,4-dimethyl-2-thioxo-chinazoliniums eingesetzt wird. Daher sind die Verbindungen dieser Gruppe besonders bevorzugt.

Das anwendungsbereite Färbemittel - beziehungsweise das Mittel A2 - enthält mindestens eine reaktive Carbonylverbindung.

Reaktive Carbonylverbindungen besitzen im Sinne der Erfindung mindestens eine Carbonylgruppe als reaktive Gruppe, welche mit der CH-aciden Komponente unter Ausbildung einer kovalenten Bindung reagiert. Bevorzugte reaktive Carbonylverbindungen sind ausgewählt aus Verbindungen, die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente

(Oxo2) stets vorhanden ist. Diese Derivate sind bevorzugt Additionsverbindungen

a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente (Oxo1) leitet sich in diesem Fall c) von einem Aldehyd ab)

an das Kohlenstoffatom der Carbonylgruppe der reaktiven Carbonylverbindung.

Unter den reaktiven Carbonylverbindungen sind Verbindungen ausgewählt aus Verbindungen der folgenden Gruppe bevorzugt: Benzaldehyd und seine Derivate, Naphthaldehyd und seine Derivate, Zimtaldehyd und seine Derivate, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, Pyridoxal, 2,3-Dihydro-1,5-dimethyl-3-oxo-2-phenyl-1H-pyrazol-4-carboxaldehyd, Furfural, 5-Nitrofurfural, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxy-phenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzyl-chinolinium-, 6-Formyl-1-benzylchinolinium-, 7-Formyl-1-benzylchinolinium-, 8-Formyl-1-benzyl-chinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat, - chlorid, -bromid, -iodid, -tetrachlorozinkat, -methylsulfat-, -trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Insbesondere bevorzugt ist es, wenn als reaktive Carbonylverbindung eine Verbindung ausgewählt aus mindestens einer Verbindungen der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxybenzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxy-benzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methylbenzaldehyd, 3,4-Dihydroxy-5-methyl-benzaldehyd, 3,4-Dihydroxy-6-methyl-benzaldehyd, 3,4-Dihydroxy-2-methoxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 3-Carboxy-4-hydroxy-benzaldehyd, 5-Carboxyvanillin, 3-Carboxy-4-hydroxy-5-methylbenzaldehyd, 3-Carboxy-5-ethoxy-4-hydroxybenzaldehyd, 3-Carboxy-4-hydroxybenzaldehyd, 5-Carboxyvanillin, 3-Carboxy-4-hydroxy-5-methylbenzaldehyd, 3-Carboxy-5-ethoxy-4-hydroxybenzaldehyd, 3-Allyl-4-hydroxybenzaldehyd, 3-Allyl-4-hydroxy-5-methoxybenzaldehyd, 3-Allyl-4-hydroxy-5-methylbenz-aldehyd, 3-Allyl-5-brom-4-hydroxybenzaldehyd, 3,5-Diallyl-4-hydroxybenzaldehyd, 3-Allyl-5-carboxy-4-hydroxybenzaldehyd (3-Allyl-5-formyl-2-hydroxybenzoesäure), 3-Allyl-4-hydroxy-5-formylbenzaldehyd, (5-Allyl-4-hydroxyisophthalaldehyd) und 2,3-Dihydro-1,5-dimethyl-3-oxo-2-phenyl-1 H-pyrazol-4-carboxaldehyd eingesetzt wird.

Die reaktiven Carbonylverbindungen werden vorzugsweise in einer Menge von 0,03 bis 65,00 mmol, bezogen auf 100 g des anwendungsbereiten Färbemittels, eingesetzt. Der Einsatz von 1,00 bis 30,00 mmol, bezogen auf 100 g des anwendungsbereiten Färbemittels, ist insbesondere bevorzugt.

Die CH-aciden Verbindungen der Formel (CH-1) und/oder der Formel (CH-2) in dem anwendungsbereiten Färbemittel werden vorzugsweise in einer Menge von 0,03 bis 65,00 mmol, bezogen auf 100 g des anwendungsbereiten Färbemittels, eingesetzt. Der Einsatz von 1,00 bis 30,00 mmol, bezogen auf 100 g des anwendungsbereiten Färbemittels, ist insbesondere bevorzugt.

Die Mittel A1 und A2 können im Rahmen einer Ausführungsform in dem erfindungsgemäßen Verfahren pulverförmig, granuliert oder als Formkörper vorliegen.

Ein pulverförmiges Mittel A1 bzw. A2 besitzt eine bevorzugte mittlere Teilchengröße von 0,0001 bis 50 μm, insbesondere von 0,05 bis 30 μm.

Als Granulate werden erfindungsgemäß körnige Partikel verstanden. Diese körnigen Partikel sind fließfähig.

Granulate können durch Feuchtgranulierung, durch Trockengranulierung bzw. Kompaktierung und durch Schmelzerstarrungsgranulierung hergestellt werden. Die gebräuchlichste Granuliertechnik ist die Feuchtgranulierung, da diese Technik den wenigsten Einschränkungen unterworfen ist und am sichersten zu Granulaten mit günstigen Eigenschaften führt. Die Feuchtgranulierung erfolgt durch Befeuchtung der Pulvermischungen mit Lösungsmitteln und/oder Lösungsmittelgemischen und/oder Lösungen von Bindemitteln und/oder Lösungen von Klebstoffen und wird vorzugsweise in Mischern, Wirbelbetten oder Sprühtürmen durchgeführt, wobei besagte Mischer beispielsweise mit Rühr- und Knetwerkzeugen ausgestattet sein können. Für die Granulation sind jedoch auch Kombinationen von Wirbelbett(en) und Mischer(n), bzw. Kombinationen verschiedener Mischer einsetzbar. Die Granulation erfolgt unter Einwirkung niedriger bis hoher Scherkräfte.

Wenn die Mittel A1 bzw. A2 als Formkörper vorliegt, dann können diese erfindungsgemäßen Formkörper jedwede geometrische Form aufweisen, wie beispielsweise konkave, konvexe, bikonkave, bikonvexe, kubische, tetragonale, orthorhombische, zylindrische, sphärische, zylindersegmentartige, scheibenförmige, tetrahedrale, dodecahedrale, octahedrale, konische, pyramidale, ellipsoide, fünf-, sieben- und achteckig-prismatische sowie rhomboedrische Formen. Auch völlig irreguläre Grundflächen wie Pfeil- oder Tierformen, Bäume, Wolken usw. können realisiert werden. Die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt und Formkörper mit sphärischer Geometrie sind erfindungsgemäß bevorzugt. Besonders bevorzugt sind Formkörper in Gestalt sphärischer Geometrie.

Die zylinderförmige Ausgestaltung erfaßt dabei die Darbietungsform von der Tablette bis zu kompakten Zylinderstücken mit einem Verhältnis von Höhe zu Durchmesser größer 1. Weist der Basisformkörper Ecken und Kanten auf, so sind diese vorzugsweise abgerundet. Als zusätzliche optische Differenzierung ist eine Ausführungsform mit abgerundeten Ecken und abgeschrägten ("angefasten") Kanten bevorzugt.

Die sphärische Ausgestaltung umfaßt neben einer kugelförmigen Gestalt auch einen Hybrid aus Kugel- und Zylinderform, wobei jede Grundfläche des Zylinders mit je einer Halbkugel überkappt ist. Die Halbkugeln haben bevorzugt einen Radius von ca. 4 mm und der gesamte Formkörper dieser Ausgestaltung eine Länge von 12 - 14 mm.

Ein erfindungsgemäßer Formkörper mit sphärischer Ausgestaltung kann nach den bekannten Verfahren hergestellt werden. Es ist dabei möglich, die Formkörper durch Extrusion eines Vorgemisches mit nachfolgender Formgebung zu produzieren.

Die als Feststoff konfektionierten Mittel A1 und/oder A2 enthalten im Rahmen einer bevorzugten Ausführungsform mindestens ein Alkalisierungsmittel.

Diese Alkalisierungsmittel werden wiederum bevorzugt aus mindestens einem Alkalisierungsmittel ausgewählt aus der Gruppe, die gebildet wird, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, Natriumhydroxid, L-Arginin, D-Arginin, DL-Arginin, N-Methylglucamin, Morpholin, Imidazol und Harnstoff.

Im Rahmen der Ausführungsform der als Feststoff vorliegenden Mittel A1 und A2 sind die als Feststoff vorliegenden Alkalisierungmittel besonders bevorzugt.

Die als Pulver, Granulat oder Formkörper vorliegenden Mittel A1 und A2 werden dann gemeinsam in einem flüssigen kosmetischen Mittel A3 oder in Wasser unter Erhalt des anwendungsbereiten Färbemittels gemischt. Das flüssige kosmetische Mittel A3 kann im Rahmen dieser Ausführungsform ebenso vom erfindungsgemäßen Verfahren umfasst sein.

Als flüssige kosmetische Träger für das Mittel A3 eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Die flüssigen kosmetischen Träger können insbesondere wässrig oder wässrig-alkoholisch sein.

Unter flüssig wird erfindungsgemäß verstanden, wenn der kosmetische Träger bei 25°C bei einem Druck von 1 atm einen flüssigen Aggregatzustand besitzt.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines Alkohols.

Es ist erfindungsgemäß bevorzugt, wenn das Mittel A3 zusätzlich mindestens ein organisches Lösemittel enthält. Dieses organische Lösemittel wird wiederum bevorzugt ausgewählt aus der Gruppe, die gebildet wird, aus

- ($C_1$ bis $C_4$)-Monohydroxyalkoholen,
- ($C_3$ bis $C_6$)-Dihydroxyalkoholen,
- ($C_3$ bis $C_6$)-Trihydroxyalkoholen,
- zyklischen, organischen Carbonaten,
- Verbindungen der Formel $H\text{-}(O\text{-}CH_2CH_2)_n\text{-}OR$ mit R = Wasserstofatom, Methylgruppe und n = 1, 2, 3 oder 4

Als bevorzugte ($C_1$ bis $C_4$)-Monohydroxyalkohole gelten Ethanol und Isopropanol.

Als bevorzugter ($C_3$ bis $C_6$)-Dihydroxyalkohol gilt 1,2-Propandiol.

Als bevorzugter ($C_3$ bis $C_6$)-Trihydroxyalkohol gilt Glyzerin.

Als zyklisches, organisches Carbonat eignet sich erfindungsgemäß bevorzugt mindestens ein zyklischer Kohlensäureester. Diese zyklischen Ester der Kohlensäure leiten sich vom 1,3-Dioxolan-2-on ab und lassen sich durch folgende Grundstruktur der Formel (I-1) beschreiben:

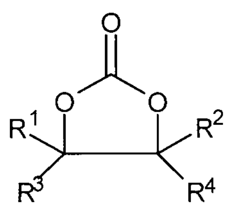

(I-1)

worin die Reste $R^1$, $R^2$, $R^3$ und $R^4$ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können.

Im Grundkörper, dem 1,3-Dioxolan-2-on, stehen die Reste $R^1$, $R^2$, $R^3$ und $R^4$ der Formel (I-1) jeweils für ein Wasserstoffatom. Weiterhin bevorzugt geeignete cyclische Kohlensäureester betreffen Derivate dieses Grundkörpers,

wobei mindestens einer der Reste $R^1$, $R^2$, $R^3$ und $R^4$ der Formel (I-1) von einem Wasserstoffatom verschieden ist. Hierbei sind der strukturellen Vielfalt keine Grenzen gesetzt, so daß sich mono-, di-, tri- und tetra-substituierte 1,3-Dioxolan-2-one der Formel (I-1) zum Einsatz im Rahmen der vorliegenden Erfindung eignen.

Besonders bevorzugt sind neben dem unsubstituierten 1,3-Dioxolan-2-on insbesondere die in 4-Stellung monosubstituierten Derivate der nachstehenden Formel (I-2)

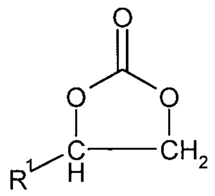

(I-2)

in der $R^1$ für einen substituierten oder unsubstituierten Alkyl-, Alkenyl- oder Alkylarylrest steht.

Bevorzugte Reste $R^1$ gemäß Formel (I-2) sind Methyl-, Ethyl-, n-Propyl-, iso-Propyl- sowie Hydroxymethyl-, 1-Hydroxyethyl- und 2-Hydroxyethyl-Reste.

Besonders bevorzugte erfindungsgemäße Mittel sind folglich dadurch gekennzeichnet, daß sie als 1,3-Dioxolan-2-on-Derivat mindestens eine Verbindung der obigen Formel (I-2) enthalten, bei der $R^1$ für einen substituierten oder unsubstituierten Alkyl-, Alkenyl- oder Alkylarylrest steht, wobei in weiter bevorzugten erfindungsgemäßen Mitteln der Rest $R^1$ in Formel (I-2) ausgewählt ist aus Methyl-, Ethyl-, n-Propyl-, iso-Propyl- sowie Hydroxymethyl-, 1-Hydroxyethyl- und 2-Hydroxyethyl-Resten.

Besonders bevorzugte 1,3-Dioxolan-2-one der Formel (I-1) stammen aus der Gruppe Ethylencarbonat ($R^1$, $R^2$, $R^3$ und $R^4$ = H), Propylencarbonat ($R^1 = CH_3$ sowie $R^2$, $R^3$ und $R^4$ = H) und Glycerincarbonat ($R^1 = CH_2OH$ sowie $R^2$, $R^3$ und $R^4$ = H). Ganz besonders bevorzugt eignet sich Propylencarbonat.

Ethylencarbonat ist eine farblose kristalline Verbindung, die bei 39°C schmilzt und bei 238°C siedet. Das in Wasser, Alkoholen und organischen Lösungsmitteln leicht lösliche Ethylencarbonat ist über großtechnische Synthesen aus Ethylenoxid und flüssigem $CO_2$ herstellbar. Propylencarbonat ist eine wasserhelle, leichtbewegliche Flüssigkeit, mit einer Dichte von 1,2057 $gcm^{-3}$, der Schmelzpunkt liegt bei -49°C, der Siedepunkt bei 242°C. Auch Propylencarbonat ist großtechnisch durch Reaktion von Propylenoxid und $CO_2$ bei 200°C und 80 bar zugänglich. Glycerincarbonat ist durch Umesterung von Ethylencarbonat oder Dimethylcarbonat mit Glycerin zugänglich, wobei als Nebenprodukte Ethylenglycol bzw. Methanol anfallen. Ein weiterer Syntheseweg geht von Glycidol (2,3-Epoxy-1-propanol) aus, das unter Druck in Gegenwart von Katalysatoren mit $CO_2$ zu Glycerincarbonat umgesetzt wird. Glycerincarbonat ist eine klare, leichtbewegliche Flüssigkeit mit einer Dichte von 1,398 $gcm^{-3}$, die bei 125-130°C (0,15 mbar) siedet.

Bevorzugte Verbindungen der Formel $H-(O-CH_2CH_2)_n-OR$ mit R = Wasserstofatom, Methylgruppe und n = 1, 2, 3 oder 4 werden ausgewählt aus Ethylenglycol, Diethylenglycol, Diethylenglycolmonomethylether.

Besonders bevorzugt ist ein Gemisch aus

Ethylenglycol und Isopropanol,
oder aus
Isopropanol, Propylencarbonat und Diethylenglycol
als organische Lösemittel zu verwenden.

Die organischen Lösemittel werden im Mittel A3 bevorzugt in einer Menge von 0,5 bis 20 Gew.-%, insbesondere von 2 bis 10 Gew.-%, ganz besonders von 3 bis 6 Gew.-%, jeweils bezogen auf das Gewicht des Mittels A3, eingesetzt.

Im Rahmen einer besonders bevorzugten Ausführungsform, liegt das Färbemittel in dem erfindungsgemäßen Verfahren in folgenden Komponenten vor:

- das Mittel A1 enthält in einem flüssigen kosmetischen Träger mindestens eine CH-acide Verbindung und weist einen pH-Wert von 0,5 bis 2,5 aufweist und
- das Mittel A2 enthält in einem flüssigen kosmetischen Träger mindestens eine reaktive Carbonylverbindung und weist einen pH-Wert von 2 bis 5 auf.

Besonders lagerstabile Formulierungen können erhalten werden, wenn das Mittel A1 einen pH-Wert von 1 bis 2 und insbesondere bevorzugt einen pH-Wert von 1,1 bis 1,9 aufweist, daher ist dieser pH-Bereich bevorzugt.

Desweiteren ist es bevorzugt, wenn das Mittel A2 einen pH-Wert von 3 bis 5, insbesondere von 3,2 bis 4,5, aufweist.

Bei den pH-Werten im Sinne der vorliegenden Erfindung handelt es sich um pH-Werte, die bei einer Temperatur

von 22 °C gemessen wurden.

Die Einstellung des pH-Wertes in den Mitteln A1 und/oder A2 kann mit Hilfe einer organischen oder anorganischen Säure wie beispielsweise Salzsäure, Schwefelsäure, Bromwasserstoffsäure, Phosphorsäure, Essigsäure, Weinsäure, Zitronensäure, Milchsäure, Äpfelsäure oder Glykolsäure erfolgen.

In diesem Zusammenhang ist die Einstellung der erfindungsgemäßen pH-Werte mit Hilfe von Salzsäure, Weinsäure, Zitronensäure, Äpfelsäure oder Milchsäure besonders bevorzugt.

Für die Anwendung im erfindungsgemäßen Verfahren können die Mittel A1 und A2 kurz vor dem Aufbringen auf die Haarfaser innig miteinander vermischt werden. Zur weiteren Verbesserung des Färbeergebnisses kann es jedoch vorteilhaft sein, die Färbung selbst in einem alkalischen pH-Bereich durchzuführen.

Daher kann im Rahmen einer besonderen Ausführungsform das erfindungsgemäßen Verfahren zusätzlich ein drittes, zuvor beschriebenes flüssiges kosmetisches Mittel A3 *(vide supra)* umfassen, welches in einem kosmetischen Träger mindestens ein Alkalisierungsmittel enthält und einen pH-Wert größer als 7 aufweist. Diese Ausführungsform des Kits ist gleichermassen für alle Ausführungsformen der Mittel A1 und A2 bevorzugt, d.h. sowohl für die Ausführungsform von A1 und A2 als Feststoff (Pulver, granulat oder Formkörper) als auch für die Ausführungsform von A1 und A2 als Flüssigkeiten mit speziellem pH-Wert.

Erfindungsgemäß sind somit solche Verfahren bevorzugt, in denen die Mittel A1, A2 und A3 so ausgestaltet sind, dass das anwendungsbereite Färbemittel einen pH-Wert größer 7 aufweist.

Die flüssigen kosmetischen Mittel A3 enthalten bevorzugt zusätzlich mindestens ein Alkalisierungsmittel.

Die im flüssigen kosmetischen Mittel A3 enthaltenen Alkalisierungsmittel werden bevorzugt aus mindestens einem Alkalisierungsmittel ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus Ammoniak, 2-Aminoethanol, 2-Amino-2-methylpropan-1-ol, 2-Amino-2-methyl-propan-1,3-diol, Kaliumhydroxid, Natriumhydroxid, L-Arginin, D-Arginin, DL-Arginin, N-Methylglucamin, Morpholin, Imidazol und Harnstoff.

Desweiteren können auch auf einen pH-Wert größer als 7 eingestellte Puffersysteme in dem flüssigen kosmetischen Mittel A3 eingesetzt werden.

Als pH-Puffersystem werden erfindungsgemäß solche chemische Verbindungen bzw. eine Kombination aus chemischen Verbindungen angesehen, die in einer Lösung bewirken, dass sich der pH-Wert der Lösung bei Zugabe einer kleinen Menge Säure oder Lauge zu einem Volumen des kosmetischen Trägers nur geringfügig ändert. Diese Änderung ist weniger ausgeprägt, als dies bei einer Zugabe der gleichen Menge an Säure oder Lauge zu einem gleichen Volumen des kosmetischen Trägers ohne pH-Puffersystem der Fall ist.

Solche pH-Puffersysteme sind bevorzugt ausgewählt aus mindestens einem Vertreter aus der Gruppe, die gebildet wird aus Hydrogencarbonat/Carbonat, Genußsäure (insbesondere Citronensäure)/Monohydrogenphosphat, Genußsäure (insbesondere Citronensäure)/Dihydrogenphosphat, Tris(hydroxymethyl)aminomethan/Maleinsäure/NaOH, Tris(hydroxymethyl)aminomethan/Maleinsäure/KOH, Tris(hydroxymethyl)aminomethan/HCl, Monohydrogenphosphat/Dihydrogenphosphat, Dihydrogenphosphat/NaOH, Dihydrogenphosphat/KOH, $H_3BO_3$/KCl/NaOH, $H_3BO_3$/KCl/KOH, Borat/HCl, Borat/Halogenid (insbesondere Chlorid wie Kaliumchlorid)/NaOH, Borat/Halogenid (insbesondere Chlorid wie Kaliumchlorid)/KOH, Puffersystem nach Theorell und Stenhagen, Puffersystem nach McIlvine, Glycin/NaOH und Glycin/KOH. Besonders bevorzugte pH-Puffersysteme werden ausgewählt aus mindestens einem Vertreter aus der Gruppe, die gebildet wird, aus Tris(hydroxymethyl)aminomethan/Maleinsäure/NaOH, Tris(hydroxymethyl)aminomethan/Maleinsäure/KOH, Tris(hydroxymethyl)aminomethan/HCl, Borat/HCl und $H_3BO_3$/KCl/NaOH.

Die mit dem Schrägstrich gekennzeichneten pH-Puffersysteme aus obiger Liste stellen Gemische dieser durch

den Schrägstrich getrennten Verbindungen dar. Die in der Liste angegebenen anionischen Verbindungen werden in Form deren Salze mit einem korrespondierenden ein- oder mehrwertigen Kation eingesetzt. Bevorzugte Kationen sind Alkalimetallkationen (insbesondere Natrium oder Kalium) und Ammoniumionen. Erfindungsgemäß in den Puffersystemen verwendbare Genußsäuren sind beispielsweise Citronensäure, Weinsäure oder Äpfelsäure bzw. deren Gemische.

Das pH-Puffersystem ist bevorzugt in einer Menge von 0,1 bis 10,0 Gew.-%, besonders bevorzugt von 0,3 bis 5,0 Gew.-%, ganz besonders bevorzugt von 0,5 bis 3,0 Gew.-%, jeweils bezogen auf das Gewicht der Anwendungsmischung aus Mittel A1 und Mittel A2, bzw. aus den Mitteln A1, A2 und A3, in dem anwendungsbereiten Färbemittel enthalten.

Der pH-Wert des anwendungsbereiten Färbemittels, welche durch Vermischen der Mittel A1, A2 und A3, hergestellt wird, liegt bevorzugt zwischen 7,5 und 11. Liegt der pH-Wert des anwendungsbereiten Färbemittels in einem Bereich von 8 bis 10, so ist dies bevorzugt.

In einer weiteren Ausführungsform des Kits kann in dem anwendungsbereiten Färbemittel bzw. in dem Mittel A1 und/oder in dem Mittel A2 zusätzlich mindestens eine Entwicklerkomponente und gegebenenfalls mindestens eine Kupplerkomponente als Oxidationsfarbstoffvorprodukte enthalten. Es ist jedoch bevorzugt, die erfindungsgemäßen Mittel frei von Oxidationsfarbstoffvorprodukten zu formulieren, insbesondere wenn das Allergierisiko minimiert werden soll. Somit kennzeichnet sich ein bevorzugtes erfindungsgemäßes Kit dadurch, daß das anwendungsbereite Färbemittel - beziehungsweise die Mittel A1 und A2 - frei von Oxidationsfarbstoffvorprodukten, insbesondere vom Entwicklertyp, sind.

Weiterhin kann in dem anwendungsbereiten Färbemittel - bzw. den Mitteln A1 und/oder A2 - zusätzlich mindestens ein direktziehender Farbstoff enthalten sein. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethyl-amino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel können die fakultativ enthaltenen direktziehenden Farbstoffe in einer Menge von 0,01 bis 20,00 Gew.-%, bezogen auf den erfindungsgemäßen Kit und damit bezogen auf das anwendungsbereite Färbemittel enthalten. Weiterhin kann der erfindungsgemäße Kit auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Das Entfärbemittel B des erfindungsgemäßen Kits enthält in einem kosmetischen Träger Wasserstoffperoxid.

Erfindungsgemäß bevorzugte Entfärbemittel B enthalten das Wasserstoffperoxid in Mengen von 0,5 und 12 Gew.-%, bevorzugt in Mengen von 1 und 6 Gew.-%, besonders bevorzugt in Mengen von 1 und 4 Gew.-%, ganz besonders bevorzugt in Mengen von 1,5 bis 3 Gew.-%, jeweils berechnet auf das Gewicht des gesamten Mittels.

Das Entfärbemittel B weist bevorzugt einen pH-Wert von 4 bis 11, besonders bevorzugt von 5 bis 10, ganz besonders bevorzugt von 7 bis 10 auf.

Darüber hinaus ist es erfindungsgemäß wenn das Entfärbemittel B neben Wasserstoffperoxid weniger als 0,001 Gew.-% weiterer Peroxoverbindungen enthält, d.h. im Sinne der Erfindung frei ist von weiteren Peroxoverbindungen. Peroxoverbindungen sind chemische Verbindungen, die die Gruppe -O-O- bzw. $O_2^{2-}$ im Molekül enthalten.

Unter die im Rahmen dieser Ausführungsform des Entfärbemittels B definierten weiteren Peroxoverbindungen fällt Wasserstoffperoxid nicht und somit auch keine Perhydrate.

Zu vermeidende Peroxoverbindungen sind organische Persäuren, Peroxidisulfatsalze, Persulfatsalze, Peroxidiphosphatsalze (insbesondere Ammoniumperoxidisulfat, Kaliumperoxidisulfat, Natriumperoxidisul¬fat, Ammoniumpersulfat, Kaliumpersulfat, Natriumpersulfat, Kaliumperoxidiphosphat) und Peroxide (wie Bariumperoxid und Magnesiumperoxid). Daher sind insbesondere solche Entfärbemittel bevorzugt, die frei sind von organischen Persäuren und Peroxidisulfatsalzen und Persulfatsalzen und Peroxidiphosphatsalzen und Peroxiden.

Es ist erfindungsgemäß bevorzugt, wenn das Entfärbemittel B zusätzlich mindestens ein Tensid enthält. In vielen Fällen enthalten die anwendungsbereiten Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den Mitteln (insbesondere A1, A2, A3, B) alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R\text{-}O(CH_2\text{-}CH_2O)_x\text{-}SO_3H$, in der R eine bevorzugt lineare Alkylgruppe mit 10 bis 18 C-Atomen und x = 0 oder 1 bis 12 ist,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, -phosphaten und/oder - isethionaten, die sich von Alkyl- und/oder Alkenyloligoglykosiden der allgemeinen Formel (II) ableiten,

$$R\text{-}O\text{-}(G)_p \qquad (II)$$

mit der Bedeutung

R $C_{6\text{-}22}$-Alkyl oder $C_{6\text{-}22}$-Alkenyl,
G Glykosideinheit, die sich von einem Zucker mit 5 oder 6 Kohlenstoffatomen ableitet,
p Zahl von 1 bis 10,

insbesondere das Laurylglucosidcarboxylat, wie es als Plantapon® LGC von Cognis Deutschland erhältlich ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 60 Mol Ethylenoxid und/oder 0 bis 5 Mol Propylenoxid an lineare Fettalkohole mit

8 bis 22 C-Atomen, an Fettsäuren mit 12 bis 22 C-Atomen und an Alkylphenole mit 8 bis 15 C-Atomen in der Alkylgruppe,

- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O$-(Z)x. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Beispiele für solche nichtionischen Tenside werden unter dem Handelsnamen Cremophor® RH40 (hydriertes Rizinusöl mit ca. 40-45 EO-Einheiten (INCI-Bezeichnung: PEG-40 Hydrogenated Castor Oil)) von der Firma BASF oder Mergital® CS 50A (Fettalkohol mit ca. 50 EO-Einheiten (INCI-Bezeichnung: Ceteareth-50) von der Firma Cognis vertrieben.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder - $SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12}$-$_{18}$-Acylsarcosin. Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Gleichfalls können selbstverständlich die Mittel A1, A2 und A3 zusätzlich mindestens ein Tensid enthalten.

Die Mittel A1, A2, A3 und/oder das Entfärbemittel B im erfindungsgemäßen Verfahren können zusätzlich mindestens ein Silikon enthalten. Die Silikone, wenn sie in den besagten Mitteln bevorzugt zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone ausgewählt, aus mindestens einem Vertreter aus der Liste, die gebildet wird aus:

(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:

a) substituierten oder unsubstituierten aminierten Gruppen;
b) (per)fluorierten Gruppen;
c) Thiolgruppen;
d) Carboxylatgruppen;
e) hydroxylierten Gruppen;

f) alkoxylierten Gruppen;
g) Acyloxyalkylgruppen;
h) amphoteren Gruppen;
i) Bisulfitgruppen;
j) Hydroxyacylaminogruppen;
k) Carboxygruppen;
l) Sulfonsäuregruppen; und
m) Sulfat- oder Thiosulfatgruppen;

(iii) linearen Polysiloxan(A)- Polyoxyalkylen(B)- Blockcopoylmeren vom Typ $(A-B)_n$ mit $n > 3$;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Zusätzlich können die Mittel A1, A2, A3 und/oder Entfärbemittel B mindestens ein Proteinhydrolysat enthalten. Die Proteinhydrolysate sind Produktgemische, die durch sauer, basisch oder enzymatisch katalysierten Abbau von Proteinen (Eiweißen) erhalten werden.
Erfindungsgemäß können Proteinhydrolysate sowohl pflanzlichen als auch tierischen Ursprungs eingesetzt werden.
Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Keratin DEC® (Vincience), Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben. Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.
Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.
Bevorzugt sind die Proteinhydrolysate in einer Menge von 0,05 bis 5 Gew.-%, besonders bevorzugt von 0,5 bis 2,0 Gew.-%, jeweils bezogen auf das Gewicht des anwendungsbereiten Färbemittels bzw. des Entfärbemittels B, enthalten.
In einer weiteren Ausführungsform des Kits können die Mittel A1, A2, A3 und/oder das Entfärbemittel B zusätzlich mindestens ein kationisches und/oder mindestens ein amphoteres Polymer enthalten.
Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert der Mittel eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (P1),

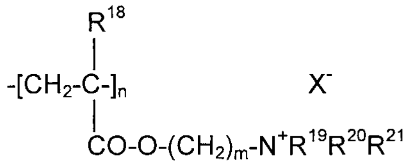 (P1)

in der $R^{18}$ = -H oder -$CH_3$ ist, $R^{19}$, $R^{20}$ und $R^{21}$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (P1) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^{18}$ der Formel (P1) steht für eine Methylgruppe
- $R^{19}$, $R^{20}$ und $R^{21}$ der Formel (P1) stehen für Methylgruppen
- m der Formel (P1) hat den Wert 2.

Als physiologisch verträgliches Gegenionen $X^-$ der Formel (P1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.

- quaternierter Polyvinylalkohol,
  sowie die unter den Bezeichnungen

  - Polyquaternium 2 (z.B. Mirapol® A-15 der Firma Rhodia),
  - Polyquaternium 17,
  - Polyquaternium 18 und
  - Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von $5 \cdot 10^5$ bis $5 \cdot 10^6$ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbohsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

- Unter dem Begriff amphotere Polymere werden solche Polymere verstanden,

  - die im Molekül sowohl freie Aminogruppen als auch freie -COOH- oder $SO_3H$-Gruppen enthalten und zur Ausbildung innerer Salze befähigt sind,
  - zwitterionische Polymere, die im Molekül quartäre Ammoniumgruppen und $-COO^-$-Gruppen oder $-SO_3^-$-Gruppen enthalten, sowie
  - Polymere, die -COOH-Gruppen oder $SO_3H$-Gruppen und quartäre Ammoniumgruppen enthalten.

Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butyl-maleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z.

B. Bentonit oder vollsynthetische Hydrokolloide wie z. B. Polyvinylalkohol,
- Strukturanten wie Glucose und Maleinsäure,
- Parfümöle, Dimethylisosorbid und Cyclodextrine,
- Antischuppenwirkstoffe wie Piroctone Olamine und Zink Omadine,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien.

Die Bestandteile des kosmetischen Trägers werden zur Herstellung der Mittel A1, A2, A3 bzw. des Entfärbemittels B in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% bezogen auf das jeweilige Mittel eingesetzt.
Es ist erfindungsgemäß bevorzugt, das Entfärbemittel 5 bis 20 Minuten auf der Faser zu belassen.

## Beispiele

Die in den Beispielen verwendeten Mengenangaben verstehen sich, sofern nicht anderes angegeben ist, in Gewichtsprozent.

### 1.0 Bereitstellung der Mittel A1

Es wurden folgende Mittel A1 gemäß Tabelle 1 bereitgestellt:

Tabelle 1

| Komponente | A1a | A1b | A1c |
|---|---|---|---|
| 1,2-Dihydro-1,3,4,6-tetramethyl-2-oxo- | 10mmol | - | - |
| 1-Allyl-1,2-dihydro-3,4,6-trimethyl-2-oxo-pyrimidiniumbromid | - | 10mmol | - |
| 2(Cyanmethyl)-benzim idazol | - | - | 10mmol |
| N-Hance® 3196 | - | - | 1,2 |
| Carbopol ETD 2020® | 1,0 | - | - |
| Xanthan Gum | - | 1,6 | - |
| Natriumbenzoat | 0,3 | - | 0,6 |
| Propylparaben | 0,15 | 0,5 | - |
| Kaliumchlorid | 0,37 | - | - |
| 0,2mol/L HCl-Lösung | 0,07 | - | - |
| Weinsäure | Ad pH | Ad pH | Ad pH |
| Wasser | Ad 100 | Ad 100 | Ad 100 |

1-Allyl-1,2-dihydro-3,4,6-trimethyl-2-oxopyrimidiniumbromid und/oder 2-(Cyanmethyl)-benzimidazol wurden in 70g 80 bis 90°C heißem Wasser unter Rühren gelöst, dann wurden die für die Einzelformulierungen notwendigen Mengenanteile der Komponenten Natriumbenzoat, Kaliumchlorid, 0,2mol/L Salzsäurelösung und/oder Propylparaben

zugegeben. Während des Abkühlens wurden die zuvor vorgequollenen Mittel Xanthan Gum, N-Hance 3196 und/oder Carbopol ETD 2020 unter weiterem Rühren hinzu gegeben. Der Quellvorgang wurde abgewartet. Zum Schluss wurde durch Zugabe von Weinsäure bzw. Monoethanolamin der pH-Wert eingestellt und mit kaltem Wasser auf 100g aufgefüllt.

**2.0 Bereitstellung der Mittel A2**

Es wurden folgende Mittel A2 gemäß Tabellen 2 und 3 bereitgestellt:

Tabelle 2

| **Komponente** | **A2a** | **A2b** | **A2c** | **A2d** |
|---|---|---|---|---|
| 3,5-Dimethoxy-4-hydroxybenzaldehyd | 10mmol | - | - | - |
| 3,4,5-Trihμydroxy-benzaldehyd | - | 10mm | - | - |
| 3,4-Dihydroxy-5-methoxybenzaldehyd | - | - | 10mmo | - |
| 4-Hydroxy-2-methoxybenzaldehyd | - | - | - | - |
| Lorol, techn. ® | 1,5 | - | 0,5 | - |
| Eumulgin B2® | 0,5 | - | 1,5 | - |
| Hydrenol D® | 3,5 | - | 3,0 | - |
| Cetiol PGL® | - | 1,0 | - | - |
| Eutanol G® | - | 0,5 | - | 1,0 |
| Cegesoft HF 62® | - | - | - | 2,5 |
| Tego Care 450® | - | - | - | 0,8 |
| Cutina GMS® | - | 3,0 | - | - |
| Eumulgin VL 75® | - | 0,8 | - | 0,4 |
| Protelan MST 35® | - | 0,5 | - | - |
| Plantacare 1200 UP® | - | - | - | 1,0 |
| Tego Betain 810® | - | 1,5 | - | - |
| Rewoteric AM C® | - | - | - | 2,0 |
| Texapon ALS Benz. ® | - | - | - | 1,5 |
| Dehyton K Cos® | - | 1,0 | - | - |
| Lamesoft PO 65® | - | 1,5 | - | - |
| Cosmedia Guar C 261® | - | 0,50 | - | - |
| Xanthan Gum | - | - | - | 0,50 |
| Alginat® | - | - | - | 1,0 |
| Tagat S® | 1,0 | - | - | - |
| Kokosfettsäuredi-ethanolamin C12-18 | - | - | 2,5 | - |
| Tensianol HK-2® | 3,0 | - | - | - |
| Hostapon SCI 85G® | - | - | 1,5 | - |
| Ethoxydiglycol | 1,0 | - | 0,5 | - |
| Triethanolamin | - | - | 2,0 | - |
| Propylencarbonat | 2,5 | - | 0,5 | - |
| Glycerin | 1,0 | 1,5 | - | 3,0 |
| Ethanol | - | 5,0 | 1,0 | 2,0 |

(fortgesetzt)

| Komponente | A2a | A2b | A2c | A2d |
|---|---|---|---|---|
| Mikrokill® | 0,3 | - | 0,5 | - |
| Eusolex232® | 0,6 | - | - | - |
| Benzophenone-4 | - | | 0,8 | |
| Parfum | 0,10 | - | 0,15 | - |
| Dermosoft 750® | - | 0,20 | - | 0,25 |
| Essigsäure | 0,25 | - | - | - |
| Natriumacetat | 0,15 | - | - | - |
| Weinsäure | Ad pH | Ad pH | Ad pH | Ad pH |
| Wasser | Ad 100 | Ad 100 | Ad 100 | Ad |

Tabelle 3

| Komponente | A2e | A2f | A2g | A2h |
|---|---|---|---|---|
| 2,3-Dihydro-1,5-dimethyl-3-oxo-2-phenyl-1H- | 10mmol, | - | - | - |
| 2,4-Dimethoxy-benzaldehyd | - | 10mmol, | - | - |
| 2-Formyl-1-methylchinolinium-p-toluolsulfonat | - | - | 10mmol | - |
| 4-Formyl-1-methylchinolinium-p-toluolsulfonat | - | - | - | 10mmol |
| Lorol, techn. ® | 1,5 | 2,0 | 0,5 | 1,0 |
| Eumulgin B3® | 0,5 | 1,0 | 1,5 | 1,0 |
| Hydrenol D® | 3,5 | 2,5 | 2,0 | 2,0 |
| Carbopol 940® | 0,5 | - | - | 1,5 |
| Polygel W400® | - | 1,35 | - | - |
| Walocel HM 4000 PA2910® | 0,2 | - | 0,95 | 0,2 |
| Soft CAT™ Polymer SL-5® | 1,0 | - | - | 0,5 |
| Tego Betain BL215® | - | 2,5 | - | - |
| Quartamin BTC-131® | 2,0 | - | - | 1,0 |
| Crodazosoft DBQ® | - | - | 1,5 | - |
| Kokosamidopropylbetain 40% | - | 3,0 | - | 0,5 |
| Protelan AGL 95® | - | - | 3,0 | 0,5 |
| Dow Corning 939® | 3,0 | - | 0,5 | - |
| Solan ELD® | - | - | - | 2,0 |
| Abil Soft AF 100® | - | 2,5 | 0,5 | - |
| Glycerin | - | 0,8 | - | 1,5 |
| Tween 80® | 2,0 | 1,0 | - | - |
| Glycerincarbonat | 1,0 | - | 1,5 | - |
| Benzylalkohol | - | - | 2,5 | 0,8 |
| Parsol SLX® | 0,3 | - | - | 0,15 |
| Uvinul T 150® | - | 0,3 | - | 0,10 |

(fortgesetzt)

| Komponente | A2e | A2f | A2g | A2h |
|---|---|---|---|---|
| EDTA | 0,25 | - | 0,6 | - |
| Benzoesäure DAB | - | 0,6 | 0,45 | - |
| Parfum | 0,10 | 0,25 | 0,15 | 0,20 |
| Weinsäure | Ad pH | Ad pH | Ad pH | Ad pH |
| Wasser | Ad 100 | Ad 100 | Ad 100 | Ad 100 |

Lorol, Eumulgin B1 bzw. Eumulgin B2, Hydrenol D, Cetiol PGL, Eutenol G, Cegesoft HF 62, Tego Care 450, Cutina GMS und/oder Eumulgin VL 75 wurden zusammen bei 80°C aufgeschmolzen. In diese Schmelze wurden die für die Einzelformulierungen notwendigen Mengenanteile der Komponenten Protelan MST 35, Plantacare 1200 UP, Tego Betain 810, Rewoteric AMC, Texapon ALS Benz, Dehyton K cos, Lamesoft PO 65, Soft CAT Polymer SL-5, Tego Betain BL 215, Quartamin BTC-131, Codazosoft BTQ, Kokosamidopropylbetain 40%, Protelan AGL 95, Tagat S, Kokosfettsäurediethanolamin C12-18, Tensianol HK-2 und/oder Hostapon SCI 85 G eingearbeitet. Zu diesem Gemisch wurden die angegebenen Mengenanteile der Carbonylverbindungen, gelöst in 80 bis 90°C heißem Wasser, hinzu gegeben. Während das Gemisch langsam unter Rühren auf Raumtemperatur abkühlte, wurden die übrigen Rezepturbestandteile Tween 80, Dow Corning 939, Solan ELD, Abil Soft AF 100, Glycerin, Glycerincarbonat, Benzylalkohol, Parsol SLX, Uvinul T 150, Benzoesäure DAB, Triethanolamin, Ethoxydiglycol, Propylencarbonat, Ethanol, Mikrokill, Eusolex 232, Benzophenone-4, Dermosoft 750, Essigsäure, Natriumacetat und/oder Parfum hinzugefügt. Die Quellmittel Carbopol 940, Polygel W400, Walocel HM 4000 PA2910, Cosmedia Guar C 261, Alginat und/oder Xanthan Gum wurden in Wasser vorgequollen und je nach Einzelformulierung hinzugefügt. Anschließend wurde der pH-Wert durch Zugabe von Weinsäure bzw. Monoethanolamin eingestellt. Zum Schluss wurde mit warmem Wasser auf 100g aufgefüllt.

### 3.0 Bereitstellung der Mittel A3

Es wurden folgende Mittel A3 gemäß Tabelle 4 durch einfaches vermischen der angegebenen Inhaltsstoffe bereitgestellt:

Tabelle 4

| Mittel | Komponente |
|---|---|
| A3a | 20g Monoethanolamin + 0,61g Tris + 0,10g |
| A3b | konzentrierte Ammoniaklösung in Wasser |
| A3c | 10%ige Natriumhydroxidlösung in Wasser |
| A3d | 10%ige Argininlösung in Wasser |
| A3e | 10%ige Kaliumhydroxidlösung in Wasser |

### 4.0 Ausfärbungen

Zur Herstellung der gebrauchsfertigen Färbemischung wurden die Mittel A1 und A2 im Gewichtsverhältnis 1:1 innig vermischt. Direkt anschließend wurde soviel von der Zubereitung A3 hinzugefügt, bis der in der folgenden Tabelle 5 angegebene pH-Wert der Mischung erreicht wurde. Das erhaltene gebrauchsfertige Färbemittel wurde im Verhältnis 3 g Farbmischung zu 1 g Haar (Kerling naturweiß) aufgebracht. Nach einer Einwirkzeit von 30 Minuten bei 32°C wurden die Strähnen mit lauwarmem Wasser ausgespült und danach im warmen Luftstrom (30 bis 40°C) getrocknet.

Tabelle 5

| Nuance | Rezepturkombination | Ausfärbe-pH-Wert |
|---|---|---|
| 1 | A1a / A2a / A3a | 8,5 |
| 2 | A1a / A2d / A3b | 7,5 |
| 3 | A1c / A2d / A3c | 8,4 |

(fortgesetzt)

| Nuance | Rezepturkombination | Ausfärbe-pH-Wert |
|---|---|---|
| 4 | A1c / A2b / A3d | 7,8 |
| 5 | A1c / A2c / A3e | 7,9 |
| 6 | A1c / A2e / A3a | 8,0 |
| 7 | A1c / A2f / A3b | 8,2 |
| 8 | A1c / A2g / A3c | 8,1 |
| 9 | A1c / A2h / A3d | 9,5 |
| 10 | A1b / A2a / A3e | 8,3 |
| 11 | A1b / A2d / A3d | 8,7 |
| 12 | A1b / A2b / A3b | 7,7 |
| 13 | A1b / A2c / A3c | 8,6 |
| 14 | A1c / A2a / A3a | 7,6 |
| 15 | A1a / A2g / A3e | 8,0 |
| 16 | A1a / A2h / A3d | 8,8 |

**5.0 Bereitstellung des Entfärbemittels B**

Es wurden folgende Mittel B gemäß Tabelle 6 bereitgestellt:

Tabelle 6

| Komponente | B1 | B2 | B3 | B4 | B5 |
|---|---|---|---|---|---|
| Wasserstoffperoxid | 3,0 | 6,0 | 12,0 | 3,0 | 3,0 |
| 4-Acetyl-1-methylpyridinium-p-toluolsulfonat | - | - | - | 1,0 | - |
| 3,4-Dihydro-2-methylisochinolinium-p-otluolsulfonat | - | - | - | - | 1,0 |
| Hydrenol D | 3,0 | 1,5 | - | 3,0 | 3,0 |
| Lorol, techn. | 1,5 | 1,0 | - | 1,5 | 1,5 |
| Eumulgin B2 | 1,0 | - | - | 1,0 | 1,0 |
| N-Hance 3196 | - | - | 1,10 | - | - |
| Jaguar C-17 | - | 0,75 | 0,75 | - | - |
| Texapon NSO | 2,5 | 3,0 | 2,0 | 2,5 | 2,0 |
| Turpinal SL | 1,1 | 1,5 | 1,8 | 1,4 | 1,6 |
| Dipicolinsäure | 0,05 | 0,10 | 0,15 | 0,10 | 0,10 |
| Dinatriumpyrophosphat | 0,03 | 0,02 | 0,04 | 0,03 | 0,02 |
| Schwefelsäure / Natronlauge | Ad pH 1,5 | Ad pH 2,6 | Ad pH 4,2 | Ad pH 3,8 | Ad pH 3,1 |
| Wasser | <--------------ad 100----------------> | | | | |

Lorol, Eumulgin B2 und/oder Hydrenol D wurden zusammen bei 80°C aufgeschmolzen. In diese Schmelze wurde Texapon NSO eingearbeitet und mit einem geringen Anteil von Wasser zu einer warmen Creme gerührt. Während des Abkühlens wurden Wasserstoffperoxid, 4-Acetyl-1-methylpyridinium-p-toluolsulfonat und/oder 3,4-Dihydro-2-methylisochinolinium-p-toluolsulfonat, die zuvor vorgequollenen Mittel Jaguar C-17 und/oder N-Hance 3196 und die Mittel Turpinal SL, Dipicolinsäure und/oder Dinatriumpyrophosphat hinzugefügt. Anschließend wurde durch Zugabe von Schwefelsäure bzw. Natronlauge der pH-Wert eingestellt. Zum Schluss wurde mit kaltem Wasser auf 100g aufgefüllt.

## 6.0 Entfärbung und farbmetrische Messungen

Bei den gefärbten Haarsträhnen wurden einen Tag nach dem Färbevorgang die CIE-Lab-Werte mit dem Gerät Texaflash DC 3881 der Firma Datacolor bestimmt. Zur Entfärbung wurden die Haarsträhnen mit einem der Entfärbungsmittel B (siehe Tabelle 7 und 8) durchfeuchtet. Das Flottenverhältnis betrug 3:1 (Entfärbemittel : Haar). Der pH-Wert der Auftragsmischung wurde mit dem Mittel A3c auf den, in den Tabellen 7 und 8 angegebenen pH-Wert, eingestellt. Das Entfärbungsmittel B wurde nach den in den Tabellen 7 bzw. 8 angegebenen Einwirkungszeiten bei 32°C vom Haar abgespült. Anschließend wurde das Haar im warmen Luftstrom (30 bis 40°C) getrocknet und mit dem Gerät Texaflash DC 3881 der Firma Datacolor zur Bestimmung der CIE-Lab-Werte farbmetrisch vermessen.

## 6.1 Berechnung der Farbdifferenzen der Entfärbung

Die Farbdifferenzen zwischen der gefärbten und der entfärbten Haarsträhne ergeben sich aus den jeweiligen CIE-Lab-Werten nach der unten angeführten Farbabstandsformel (siehe auch DIN 6174, Deutsche Normen, Benth Verlag GmbH, Berlin, 1975).

$$\Delta E = \left[(\Delta L)^2 + (\Delta a)^2 + (\Delta b)^2\right]^{\frac{1}{2}} \qquad \text{(Farbabstandsformel)}$$

In den Tabellen 7 und 8 sind die gemessenen CIE-Lab-Werte und der berechnete Farbabstand der erfindungsgemäßen Färbe-Entfärbe-Versuche nach dem erfindungsgemäßen Verfahren zusammengefasst. Je größer der ΔE-Wert, umso besser ist das Entfärbungsergebnis.

Tabelle 7

| | Entfärbung | pH-Wert | EWZ* | dE* | L* | a* | b* | C* | h* |
|---|---|---|---|---|---|---|---|---|---|
| keine, | keine | - | - | - | 75,01 | 2,13 | 21,73 | 21,84 | 84,30 |
| Nuance 3 | keine | - | - | - | 23,26 | 24,02 | 11,60 | 26,68 | 25,65 |
| Nuance 3 | B1 | 4,0 | 20 | 23,67 | 33,44 | 32,19 | 26,60 | 41,76 | 39,57 |
| | | 4,0 | 30 | 24,42 | 35,37 | 32,44 | 27,81 | 42,73 | 40,61 |
| Nuance 3 | B2 | 3,8 | 10 | 20,79 | 33,18 | 31,83 | 25,99 | 41,09 | 39,23 |
| | | 3,8 | 20 | 27,34 | 38,61 | 32,21 | 28,78 | 43,20 | 41,78 |
| | | 3,8 | 30 | 26,81 | 40,43 | 30,28 | 28,86 | 41,84 | 43,63 |
| Nuance 3 | B3 | 4,2 | 10 | 23,69 | 38,74 | 32,84 | 30,66 | 44,92 | 43,03 |
| | | 4,2 | 20 | 26,74 | 44,40 | 28,77 | 28,47 | 40,48 | 44,69 |
| Nuance 3 | B4 | 3,9 | 20 | 15,59 | 35,80 | 32,11 | 25,71 | 41,13 | 38,68 |
| | | 3,9 | 30 | 17,33 | 36,09 | 31,77 | 26,27 | 41,22 | 39,60 |
| Nuance 3 | B5 | 4,1 | 20 | 16,03 | 34,01 | 32,16 | 24,83 | 40,63 | 37,66 |
| | | 4,1 | 30 | 17,50 | 34,92 | 31,71 | 24,74 | 40,22 | 37,95 |
| * EWZ = Einwirkzeit des Entfärbemittels B | | | | | | | | | |

Tabelle 8*

| Nuance | | dE* | L* | a* | b* | C* | h* |
|---|---|---|---|---|---|---|---|
| 1 | coloriert | - | 19,92 | 3,44 | 0,64 | 3,49 | 10,50 |
| | entfärbt mit B1 bei pH 4,2 | 27,92 | 42,04 | 7,08 | 17,29 | 18,68 | 67,73 |
| 2 | coloriert | - | 27,18 | 30,78 | 17,78 | 35,54 | 30,02 |
| | entfärbt mit B1 bei pH 4,1 | 18,24 | 40,03 | 32,99 | 30,54 | 44,96 | 42,79 |

(fortgesetzt)

| Nuance | | dE* | L* | a* | b* | C* | h* |
|---|---|---|---|---|---|---|---|
| 3 | coloriert | - | 23,71 | 27,22 | 13,65 | 30,45 | 26,62 |
| | entfärbt mit B1 bei pH 4,0 | 14,88 | 33,53 | 31,49 | 23,98 | 39,58 | 37,29 |
| 4 | coloriert | - | 16,71 | 1,52 | 0,41 | 1,57 | 14,90 |
| | entfärbt mit B1 bei pH 3,9 | 11,38 | 26,23 | 2,90 | 6,49 | 7,10 | 65,94 |
| 5 | coloriert | - | 16,38 | 2,19 | 0,50 | 2,24 | 12,93 |
| | entfärbt mit B1 bei pH 3,8 | 12,90 | 27,19 | 5,09 | 6,92 | 8,59 | 53,65 |
| 6 | coloriert | - | 61,81 | 13,50 | 58,75 | 60,28 | 77,00 |
| | entfärbt mit B1 bei pH 3,7 | 12,99 | 66,57 | 10,51 | 47,04 | 48,20 | 77,41 |
| 7 | coloriert | - | 45,55 | 32,97 | 46,01 | 56,60 | 54,37 |
| | entfärbt mit B1 bei pH 4,3 | 20,20 | 56,41 | 19,36 | 35,76 | 40,66 | 61,58 |
| 8 | coloriert | - | 25,68 | 7,71 | 20,7 | 7,98 | 15,01 |
| | entfärbt mit B1 bei pH 3,8 | 11,38 | 35,70 | 11,79 | 5,59 | 13,05 | 25,36 |
| 9 | coloriert | - | 36,02 | 9,86 | 13,38 | 16,62 | 53,61 |
| | entfärbt mit B1 bei pH 3,9 | 11,73 | 45,28 | 10,92 | 20,50 | 23,22 | 61,95 |
| 10 | coloriert | - | 65,35 | 10,15 | 59,92 | 60,77 | 80,38 |
| | entfärbt mit B1 bei pH 4,0 | 11,81 | 68,89 | 5,90 | 49,49 | 49,84 | 83,20 |
| 11 | coloriert | - | 66,70 | 0,11 | 51,12 | 51,12 | 89,87 |
| | entfärbt mit B1 bei pH 4,1 | 2,83 | 68,17 | -1,94 | 52,40 | 52,44 | 92,12 |
| 12 | coloriert | - | 63,68 | 8,83 | 56,44 | 57,12 | 81,11 |
| | entfärbt mit B1 bei pH 4,2 | 10,62 | 66,52 | 4,82 | 47,02 | 84,15 | 10,62 |
| 3 | coloriert | - | 25,70 | 29,04 | 15,48 | 32,91 | 28,06 |
| | entfärbt mit B1 bei pH 6,8 | 3,95 | 29,39 | 30,07 | 16,45 | 34,27 | 28,69 |
| | coloriert | - | 23,53 | 26,61 | 12,80 | 29,53 | 25,70 |
| | entfärbt mit B1 bei pH 9,9 | 35,26 | 56,47 | 14,89 | 8,29 | 17,04 | 29,11 |
| | coloriert | - | 23,04 | 25,18 | 11,54 | 27,70 | 24,60 |
| | entfärbt mit B4 bei pH 10,0 | 31,36 | 51,59 | 15,40 | 20,04 | 25,28 | 52,46 |
| | coloriert | - | 25,04 | 28,72 | 14,71 | 32,27 | 27,12 |
| 3 | entfärbt mit B5 bei pH 10,1 | 26,27 | 50,10 | 21,98 | 10,61 | 24,41 | 25,77 |
| 14 | coloriert | - | 19,20 | 2,38 | -4,92 | 5,47 | 295,8 3 |
| | entfärbt mit B1 bei pH 6,8 | 42,20 | 56,84 | 3,32 | 14,13 | 14,52 | 76,79 |
| | coloriert | - | 17,89 | 2,54 | -3,92 | 4,67 | 303,0 1 |
| | entfärbt mit B1 bei pH 9,9 | 52,17 | 63,87 | -0,96 | 20,48 | 20,50 | 92,67 |
| | coloriert | - | 18,20 | 2,42 | -3,23 | 4,04 | 306,9 0 |
| | entfärbt mit B4 bei pH 10,0 | 49,81 | 60,70 | 1,36 | 22,72 | 22,76 | 86,58 |
| | coloriert | - | 18,01 | 2,40 | -3,39 | 4,16 | 305,3 5 |
| | entfärbt mit B5 bei pH 10,1 | 50,15 | 63,62 | -1,78 | 17,01 | 17,11 | 95,97 |

(fortgesetzt)

| Nuance | | dE* | L* | a* | b* | C* | h* |
|---|---|---|---|---|---|---|---|
| **5** | coloriert | - | 16,79 | 1,86 | -0,93 | 2,08 | 333,5 9 |
| | entfärbt mit B1 bei pH 7,0 | 19,95 | 36,60 | 0,91 | 1,26 | 1,55 | 54,21 |
| | coloriert | - | 16,78 | 2,28 | -1,44 | 2,70 | 327,7 0 |
| | entfärbt mit B1 bei pH 10,0 | 29,68 | 45,14 | 5,18 | 6,80 | 8,55 | 52,72 |
| **6** | coloriert | - | 66,97 | 8,36 | 53,21 | 53,86 | 81,07 |
| | entfärbt mit B1 bei pH 7,3 | 5,30 | 70,83 | 7,81 | 49,62 | 50,23 | 81,06 |
| | coloriert | - | 63,45 | 8,70 | 50,42 | 51,16 | 80,21 |
| | entfärbt mit B1 bei pH 10,2 | 28,47 | 72,28 | 2,23 | 24,13 | 24,23 | 84,71 |
| **7** | coloriert | - | 43,11 | 34,00 | 42,54 | 54,46 | 51,37 |
| | entfärbt mit B1 bei pH 7,0 | 16,12 | 54,28 | 22,38 | 43,00 | 48,48 | 62,51 |
| | coloriert | - | 43,99 | 31,73 | 43,57 | 53,90 | 53,94 |
| | entfärbt mit B1 bei pH 9,8 | 41,82 | 69,89 | 4,16 | 25,75 | 26,08 | 80,83 |
| **15** | coloriert | - | 31,67 | 8,84 | 8,61 | 12,34 | 44,26 |
| | entfärbt mit B1 bei pH 7,5 | 9,05 | 40,56 | 10,35 | 9,36 | 13,95 | 42,13 |
| | coloriert | - | 31,75 | 9,02 | 8,49 | 12,38 | 43,27 |
| | entfärbt mit B1 bei pH 10,5 | 21,52 | 53,17 | 7,91 | 6,76 | 10,41 | 40,51 |
| **16** | coloriert | - | 40,22 | 12,77 | 18,52 | 22,50 | 55,41 |
| | entfärbt mit B1 bei pH 6,5 | 14,88 | 52,92 | 10,75 | 26,02 | 28,15 | 67,55 |
| | coloriert | - | 43,28 | 13,09 | 18,12 | 22,35 | 54,14 |
| **16** | entfärbt mit B1 bei pH 10,3 | 21,02 | 61,63 | 6,90 | 26,27 | 27,16 | 75,29 |
| **13** | coloriert | - | 46,66 | 15,68 | 45,80 | 48,41 | 71,10 |
| | entfärbt mit B1 bei pH 6,7 | 2,81 | 49,07 | 16,07 | 47,19 | 49,85 | 71,19 |
| | coloriert | - | 46,16 | 16,99 | 47,10 | 50,07 | 70,16 |
| | entfärbt mit B1 bei pH 9,7 | 9,31 | 44,62 | 25,98 | 49,00 | 55,46 | 62,07 |
| *Einwirkzeit des Entfärbemittels B jeweils 20 Min | | | | | | | |

7.0 Verzeichnis der eingesetzten Rohstoffe

| | |
|---|---|
| Abil Soft® AF 100 | neutralisiertes Polyetheraminosiloxan (INCI-Bezeichnung: Methoxy PEG/PPG-7/3 Aminopropyl Dimethicone) (Evonik Degussa) |
| Carbopol® 940 | Polyarcylsäure (INCI-Bezeichnung: Carbomer) (Noveon) |
| Carbopol® ETD 2020 | vernetztes Acrylsäurecopolymer, weißes Pulver (INCI-Bezeichnung: Acrylates / C10-30 Alkylacrylate Crosspolymer) (Noveon) |
| Cegesoft® HF 62 | INCI-Bezeichnung: Hydrogenated Vegetable Oil (Cognis) |
| Cetiol® PGL | INCI-Bezeichnung: Hexyldecanol, Hexyldecyl Laurate (Cognis) |
| Cosmedia Guar® C 261 | Guarhydroxypropyltrimethylammoniumchlorid (mind. 93% Festkörper; INCI-Bezeichnung: Guar Hydroxypropyltrimonium Chloride) (Cognis CorporationCosmedia) |
| Crodazosoft® DBQ | INCI-Bezeichnung: Quaternium-91, Cetrimonium Methosulfate, Cetearyl Alcohol (ca. 70 Gew.-% kationische Aktivsubstanz) |
| Cutina® GMS | Glycerylmonostearat (max. 2% Wasser; INCI-Bezeichnung: Glyceryl Stearate) (Cognis) |

(fortgesetzt)

| | |
|---|---|
| Dehyton® K | N,N-Dimethyl-N-(C8-18-kokosamidopropyl)ammoniumaceto-betain (ca. 30% Aktivsubstanz; INCI-Bezeichnung: Aqua (Water), Cocamidopropyl Betaine) (Cognis) |
| Dermosoft® 750 | INCI-Bezeichnung: Parfum (Geranic Acid) (Dr. Straetmanns Chemische Produkte) |
| Eumulgin® B2 | Cetylstearylalkohol mit ca. 20 EO-Einheiten (INCI-Bezeichnung: Ceteareth-20) (Cognis) |
| Eumulgin® B3 | Cetylstearylalkohol mit ca. 30 EO-Einheiten (INCI-Bezeichnung: Ceteareth-30) (Cognis) |
| Eumulgin® VL 75 | Gemisch aus 20-40 Gew.-% Laurylglucosid, 20-40 Gew.-% Polyglyceryl-2 Dipolyhydroxystearat, 10-30 Gew.-% Glyzerin und 10-30 Gew.-% Wasser (INCI-Bezeichnung: Lauryl Glucoside, Polyglyceryl-2 Dipolyhydroxystearate, Glycerin) (Cognis) |
| Eusolex® 232 | 2-Phanylbenzimidazol-5-sulfonsäure (INCI-Bezeichnung: Phenylbenzimidazole Sulfonic Acid) (Merck) |
| Eutanol® G | 2-Octyldodecylalkohol (INCI-Bezeichnung: Octyldodecanol) (Cognis) |
| Hostapon® SCI 85 G | Natrium Cocoylisethionat (mindestens 84 Gew.-% Aktivsubstanz, 5-10 Gew.-% freie Fettsäure, maximal 4 Gew.-% Natriumisethionat; INCI-Bezeichnung: Sodium Cocoyl Isethionate) (Clariant) |
| Hydrenol® D | $C_{16-18}$-Fettalkohol (INCI-Bezeichnung: Cetearyl alcohol) (Cognis) |
| Jaguar® C 17 | Guar 2-hydroxy-3-trimethylammoniopropylether chlorid (INCI-Bezeichnung: Guar Hydroxypropyltrimonium Chloride) (Rhodia) |
| Lamesoft® PO65 | Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis) |
| Lorol® tech. | $C_{12-18}$-Fettalkohol (INCI-Bezeichnung: Coconut alcohol) (Cognis) |
| Mikrokill® | INCI-Bezeichnung: Polyaminopropyl Biguanide (S&D Chesham) |
| N-Hance® 3196 | Guar 2-hydroxy-3-trimethylammoniopropylether chlorid (INCI-Bezeichnung: Guar Hydroxypropyltrimonium Chloride) (Hercules) |
| Parsol® SLX | Dimethicodiethylbenzal malonat (INCI-Bezeichnung: Polysilicone-15, CAS-Nr.: 207574-74-1) (DSM Nutritional Products) |
| Plantacare® 1200 UP | $C_{12-16}$-Fettalkohol-1.4-glucosid (ca. 50-53% Aktivsubstanzgehalt; INCI-Bezeichnung: Lauryl Glucoside, Aqua (Water)) (Cognis) |
| Polygel® W 400 | Copolymer aus Ethylacrylat und Methacrylsäure in wässriger Emulsion (29-31 Gew.-% Aktivsubstanz, INCI-Bezeichnung: Acrylates Copolymer) (3V Sigma) |
| Protelan® AGL 95 | Natriumdodecylglutamat (wässrige Lösung mit ca. 33 Gew.-% Aktivsubstanz, INCI-Bezeichnung: Sodium Lauroyl Glutamate) (Zschimmer & Schwarz) |
| Protelan® MST 35 | Gemisch aus Natrium N-Myristoylsarcosinate und Natrium N-Methyl-N-cocoyltaurate (40 Gew.-% Aktivsubstanz, Zschimmer & Schwartz) |
| Quartamin® BTC-131 | ca. 50% QAV, INCI-Bezeichnung: Behenoyl PG-Trimonium Chloride (Kao) |
| Rewoteric® AM C | Natriumcocoamphoacetat (ca. 30 Gew.-% Aktivsubstanz, INCI-Bezeichnung: Sodium Cocoamphoacetate) (Evonik Degussa) |
| Soft CAT Polymer SL-5® | Cellulose mit 2-Hydroxyethylethergruppen, 2-[2-Hydroxy-3-(trimethylammonio)propoxy]ethylethergruppen und 2-Hydroxy-3-(trimethylammonio)propylethergruppen, Gegenion ist Chlorid (Feststoff, Aktivsubstanz ca. 91 Gew.-%, INCI-Bezeichnung: Polyquatemium-67) (Amerchol (Dow)) |
| Tagat S® | Ethoxyliertes Glycerylmonostearat (wachsartig (Schmelzbereich 15-25°C), HLB 16.4, INCI-Bezeichnung: PEG-30 Glyceryl Stearate) (Evonik Degussa) |
| Tego Betain® 801 | ($C_8$-$C_{10}$)-Fettsäureamidopropylbetain (Aktivsubstanz ca. 35 Gew.-%, INCI-Bezeichnung: Capryl/Capramidopropyl Beatine) (Goldschmidt) |
| Tego Betain® BL 215 | N,N-Dimethyl-N-(cocosalkylamidopropyl)ammonium acetobetain (Aktivsubstanz ca. 30 Gew.-%, INCI-Bezeichnung: Cocamidopropyl Betaine) (Goldschmidt) |
| Tego Care® 450 | Stearylglucosid (HLB 11, INCI-Bezeichnung: Polyglyceryl-3 Methylglucose Distearate) (Goldschmidt) |
| Texapon® ALS Benz. | Ammonium ($C_{12}$-$C_{16}$)-Fettalkoholsulfat (27-28 Gew.-% Aktivsubstanz, INCI-Bezeichnung: Ammonium Lauryl Sulfate) (Cognis) |
| Texapon® NSO | Laurylethersulfat, Natriumsalz (ca. 27,5% Aktivsubstanz; INCI-Bezeichnung: Sodium Laureth Sulfate) (Cognis) |

(fortgesetzt)

| | |
|---|---|
| Turpinal® SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| Tween® 80 | Sorbitanmonooleat mit ca. 20 EO-Einheiten (max. 3Gew.-% Wasser; INCI-Bezeichnung: Polysorbate-80) (Croda) |
| Uvinul® T 150 | 2,4,6-Trianilino-p-(carbo-2'-ethylhexyl-1'-oxy)-1,3,5-triazin (INCI-Bezeichnung: Ethylhexyl Triazone) (BASF) |
| Walocel® HM 4000 PA2910 | INCI-Bezeichnung: Hydroxypropyl Methylcellulose (7-12% Hydroxypropoxylgruppen, 28-30% Methoxylgruppen) (Dow) |

**Patentansprüche**

1. Verfahren zum Entfärben gefärbter keratinhaltiger Fasern, insbesondere menschlicher Haare, **dadurch gekennzeichnet, dass**

   - auf die mit einem Färbemittel, enthaltend in einem kosmetischen Träger eine Kombination aus mindestens einer CH-aciden Verbindung, die aus mindestens einer Verbindung der Formel (CH-1) und/oder aus mindestens einer Verbindung der Formel (CH-2) ausgewählt wird,

   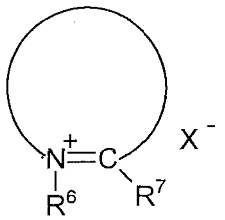 (CH-1) 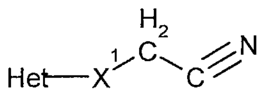 (CH-2)

   worin

   • $R^6$ steht für eine lineare oder cyclische ($C_1$ bis $C_6$)-Alkylgruppe, eine ($C_2$ bis $C_6$)-Alkenylgruppe, eine gegebenenfalls substituierte Arylgruppe, eine gegebenenfalls substituierte Heteroarylgruppe, eine Aryl-($C_1$ bis $C_6$)-alkylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxy-($C_1$ bis $C_6$)-alkylgruppe, eine Gruppe $R^IR^{II}N-(CH_2)_m-$, worin $R^I$ und $R^{II}$ stehen unabhängig voneinander für ein Wasserstoffatom, eine ($C_1$ bis $C_4$)-Alkylgruppe, eine ($C_1$ bis $C_4$)-Hydroxy-alkylgruppe oder eine Aryl-($C_1$ bis $C_6$)-alkylgruppe, wobei $R^I$ und $R^{II}$ gemeinsam mit dem Stickstoffatom einen 5-, 6- oder 7-gliedrigen Ring bilden können und m steht für eine Zahl 2, 3, 4, 5 oder 6,
   • $R^7$ steht für eine ($C_1$ bis $C_6$)-Alkylgruppe, insbesondere für eine Methylgruppe,
   • $X^-$ steht für ein physiologisch verträgliches Anion,
   • der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
   • Het steht für einen gegebenenfalls substituierten Heteroaromaten,
   • $X^1$ steht für eine direkte Bindung oder eine Carbonylgruppe,

   mit mindestens einer reaktiven Carbonylverbindung, gefärbten keratinhaltigen Fasern ein Entfärbemittel, enthaltend in einem kosmetischen Träger Wasserstoffperoxid, aufgebracht, 1 bis 30 Minuten auf der Faser belassen und anschießend wieder ausgespült wird, und
   - das Entfärbemittel frei von organischen Peroxoverbindungen und von anorganischen Persalzen ist.

2. Verfahren nach Anspruch 1, **dadurch gekennzeichnet, dass** das Entfärbemittel das Wasserstoffperoxid in Mengen zwischen 0,5 und 12 Gew.-%, bevorzugt in Mengen zwischen 0,5 und 6 Gew.-%, besonders bevorzugt in Mengen zwischen 0,5 und 4 Gew.-%, ganz besonders bevorzugt in Mengen von 0,5 bis 3 Gew.-%, jeweils berechnet auf das Gewicht des gesamten Mittels, enthält.

3. Verfahren nach Anspruch 1 oder 2, **dadurch gekennzeichnet, dass** das Entfärbemittel 5 bis 20 Minuten auf der

Faser belassen wird.

4. Verfahren nach einem der Ansprüche 1 bis 3, dadurch gekennzeichnt, dass die CH-acide Verbindung ausgewählt wird aus mindestens einer Verbindung der Gruppe bestehend aus 2-(2-Furoyl)-acetonitril, 2-(5-Brom-2-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 2-(6-Hydroxy-4,7-dimethoxy-1-benzofuran-5-oyl)-acetonitril und den Salzen mit physiologisch verträglichem Gegenion $X^-$ des 1,2-Dihydro-1,3,4,6-tetramethyl-2-oxo-pyrimidiniums, 1,2-Dihydro-1,3-diethyl-4,6-dimethyl-2-oxo-pyrimidiniums, 1,2-Dihydro-1,3-dipropyl-4,6-dimethyl-2-oxo-pyrimidiniums, 1,2-Dihydro-1,3-di(2-hydroxyethyl)-4,6-dimethyl-2-oxo-pyrimidiniums, 1,2-Dihydro-1,3-diphenyl-4,6-dimethyl-2-oxo-pyrimidiniums, 1,2-Dihydro-1,3,4-trimethyl-2-oxo-pyrimidiniums, 1,2-Dihydro-1,3-diethyl-4-methyl-2-oxo-pyrimidiniums, 1,2-Dihydro-1,3-dipropyl-4-methyl-2-oxo-pyrimidiniums, 1,2-Dihydro-1,3-di(2-hydroxyethyl)-4-methyl-2-oxo-pyrimidiniums, 1,2-Dihydro-1,3-diphenyl-4-methyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums, 1,2-Dihydro-1,3,4,6-tetramethyl-2-thioxo-pyrimidiniums, 1,2-Dihydro-1,3-diethyl-4,6-dimethyl-2-thioxo-pyrimidiniums, 1,2-Dihydro-1,3-dipropyl-4,6-dimethyl-2-thioxo-pyrimidiniums, 1,2-Dihydro-1,3-di(2-hydroxyethyl)-4,6-dimethyl-2-thioxo-pyrimidiniums, 1,2-Dihydro-1,3-diphenyl-4,6-dimethyl-2-thioxo-pyrimidiniums, 1,2-Dihydro-1,3,4-trimethyl-2-thioxo-pyrimidiniums, 1,2-Dihydro-1,3-diethyl-4-methyl-2-thioxo-pyrimidiniums, 1,2-Dihydro-1,3-dipropyl-4-methyl-2-thioxo-pyrimidiniums, 1,2-Dihydro-1,3-di(2-hydroxyethyl)-4-methyl-2-thioxo-pyrimidiniums, 1,2-Dihydro-1,3-diphenyl-4-methyl-2-thioxo-pyrimidiniums, 1,2-Dihydro-3,4-dimethyl-2-oxo-chinazoliniums und 1,2-Dihydro-3,4-dimethyl-2-thioxo-chinazoliniums.

5. Verfahren nach einem der Ansprüche 1 bis 4, **dadurch gekennzeichnet, dass** die reaktive Carbonylverbindung ausgewählt wird aus mindestens einer Verbindung der Gruppe bestehend aus Benzaldehyd und seinen Derivaten, Naphthaldehyd und seinen Derivaten, Zimtaldehyd und seinen Derivaten, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, Pyridoxal, 2,3-Dihydro-1,5-dimethyl-3-oxo-2-phenyl-1H-pyrazol-4-carboxaldehyd, Furfural, 5-Nitrofurfural, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxy-phenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat, -chlorid, - bromid, -iodid, -tetrachlorozinkat, -methylsulfat-, -trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

6. Verfahren nach einem der Ansprüche 1 bis 5 , **dadurch gekennzeichnet, daß** das Entfärbemittel B einen pH-Wert von 4 bis 11, besonders bevorzugt von 5 bis 10, ganz besonders bevorzugt von 7 bis 10 aufweist.

## Claims

1. A method for bleaching dyed keratin-containing fibers, in particular human hair, **characterized in that**

   - a bleaching agent containing hydrogen peroxide in a cosmetic carrier is applied to the keratin-containing fibers, left on the fibers for 1 to 30 minutes and then rinsed out again, which fibers have been dyed using a dye that

contains, in a cosmetic carrier, a combination of at least one CH-acidic compound, which is selected from at least one compound of the formula (CH-1) and/or from at least one compound of the formula (CH-2),

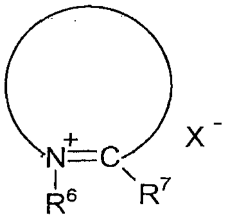 (CH-1) 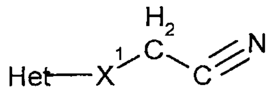 (CH-2)

in which

- $R^6$ represents a linear or cyclic ($C_1$ to $C_6$) alkyl group, a ($C_2$ to $C_6$) alkenyl group, an optionally substituted aryl group, an optionally substituted heteroaryl group, an aryl-($C_1$ to $C_6$) alkyl group, a ($C_1$ to $C_6$) hydroxyalkyl group, a ($C_2$ to $C_6$) polyhydroxyalkyl group, a ($C_1$ to $C_6$)-alkoxy-($C_1$ to $C_6$) alkyl group, a group $R^IR^{II}N-(CH_2)_m-$, in which $R^I$ and R" represent, independently of one another, a hydrogen atom, a ($C_1$ to $C_4$) alkyl group, a ($C_1$ to $C_4$) hydroxyalkyl group or an aryl-($C_1$ to $C_6$) alkyl group, in which $R^I$ and $R^{II}$ can form a 5-, 6- or 7-membered ring together with the nitrogen atom, and m represents the integer 2, 3, 4, 5 or 6,
- $\mu R^7$ represents a ($C_1$ to $C_6$) alkyl group, in particular a methyl group,
- $X^-$ represents a physiologically acceptable anion,
- the cycle of the formula (CH-1) represents all ring structures which can also contain additional heteroatoms such as nitrogen, oxygen or sulfur and can also carry uncondensed ring structures, all said ring structures being able to carry additional substituents,
- Het represents an optionally substituted heteroaromatic compound,
- $X^1$ represents a direct bond or a carbonyl group, and

at least one reactive carbonyl compound, and
- the bleaching agent is free of organic peroxo compounds and inorganic persalts.

2. The method according to claim 1, **characterized in that** the bleaching agent contains the hydrogen peroxide in amounts of between 0.5 and 12 wt.%, preferably in amounts of between 0.5 and 6 wt.%, particularly preferably in amounts of between 0.5 and 4 wt.%, more particularly preferably in amounts of from 0.5 to 3 wt.%, calculated on the basis of the weight of the overall agent in each case.

3. The method according to claim 1 or 2, **characterized in that** the bleaching agent is left on the fibers for between 5 and 20 minutes.

4. The method according to one of claims 1 to 3, **characterized in that** the CH-acidic compound is selected from at least one compound of the group consisting of 2-(2-furoyl)-acetonitrile, 2-(5-bromo-2-furoyl)-acetonitrile, 3-(2,5-dimethyl-3-furyl)-3-oxopropanenitrile, 2-(2-thenoyl)-acetonitrile, 2-(3-thenoyl)-acetonitrile, 2-(5-fluoro-2-thenoyl)-acetonitrile, 2-(5-chloro-2-thenoyl)-acetonitrile, 2-(5-bromo-2-thenoyl)-acetonitrile, 2-(5-methyl-2-thenoyl)-acetonitrile, 2-(2,5-dimethylpyrrol-3-oyl)-acetonitrile, 2-(1,2,5-trimethylpyrrol-3-oyl)-acetonitrile, 1*H*-benzimidazol-2-ylacetonitrile, 1*H*-benzothiazol-2-ylacetonitrile, 2-(pyrid-2-yl)-acetonitrile, 2,6-bis(cyanomethyl)-pyridine, 2-(indol-3-oyl)-acetonitrile, 2-(2-methyl-indol-3-yl)-acetonitrile, 2-(6-hydroxy-4,7-dimethoxy-1-benzofuran-5-oyl)-acetonitrile and the salts having the physiologically acceptable counterion $X^-$of 1,2-dihydro-1,3,4,6-tetramethyl-2-oxo-pyrimidinium, 1,2-dihydro-1,3-diethyl-4,6-dimethyl-2-oxo-pyrimidinium, 1,2-dihydro-1,3-dipropyl-4,6-dimethyl-2-oxo-pyrimidinium, 1,2-dihydro-1,3-di(2-hydroxyethyl)-4,6-dimethyl-2-oxo-pyrimidinium, 1,2-dihydro-1,3-diphenyl-4,6-dimethyl-2-oxo-pyrimidinium, 1,2-dihydro-1,3,4-trimethyl-2-oxo-pyrimidinium, 1,2-dihydro-1,3-diethyl-4-methyl-2-oxo-pyrimidinium, 1,2-dihydro-1,3-dipropyl-4-methyl-2-oxo-pyrimidinium, 1,2-dihydro-1,3-di(2-hydroxyethyl)-4-methyl-2-oxo-pyrimidinium, 1,2-dihydro-1,3-diphenyl-4-methyl-2-oxo-pyrimidinium, 1-allyl-1,2-dihydro-3,4,6-trimethyl-2-oxo-pyrimidinium, 1,2-dihydro-1-(2-hydroxyethyl)-3,4,6-trimethyl-2-oxo-pyrimidinium, 1,2-dihydro-1,3,4,6-tetramethyl-2-thioxo-pyrimidinium, 1,2-dihydro-1,3-diethyl-4,6-dimethyl-2-thioxo-pyrimidinium, 1,2-dihydro-1,3-dipropyl-4,6-dimethyl-2-thioxo-pyrimidinium, 1,2-dihydro-1,3-di(2-hydroxyethyl)-4,6-dimethyl-2-thioxo-pyrimidinium, 1,2-dihydro-1,3-diphenyl-4,6-dimethyl-2-thioxo-pyrimidinium, 1,2-dihydro-1,3,4-trimethyl-2-thioxo-pyrimidinium, 1,2-dihydro-1,3-diethyl-4-methyl-2-thioxo-pyrimidinium, 1,2-dihydro-1,3-dipropyl-4-methyl-2-thioxo-pyrimidinium, 1,2-dihydro-1,3-di(2-hydroxyethyl)-4-methyl-2-thioxo-pyrimidinium, 1,2-dihydro-1,3-diphenyl-4-methyl-2-thioxo-pyrimidinium, 1,2-dihydro-3,4-dimethyl-2-oxo-quinazolinium and 1,2-dihydro-3,4-dimethyl-2-thioxo-quinazolinium.

5. The method according to one of claims 1 to 4, **characterized in that** the reactive carbonyl compound is selected from at least one compound of the group consisting of benzaldehyde and the derivatives thereof, naphthaldehyde and the derivatives thereof, cinnamaldehyde and the derivatives thereof, 2-formylmethylene-1,3,3-trimethylindoline (Fischer's aldehyde or Tribasen aldehyde), 2-indole aldehyde, 3-indole aldehyde, 1-methylindole-3-aldehyde, 2-methylindole-3-aldehyde, 2-(1',3',3'-trimethyl-2-indolinylidene)-acetaldehyde, 1-methylpyrrole-2-aldehyde, pyridoxal, 2,3-dihydro-1,5-dimethyl-3-oxo-2-phenyl-1 H-pyrazole-4-carboxaldehyde, furfural, 5-nitrofurfural, chromone-3-aldehyde, 3-(5'-nitro-2'-furyl)-acrolein, 3-(2'-furyl)-acrolein and imidazole-2-aldehyde, 5-(4-dimethylaminophenyl)penta-2,4-dienal, 5-(4-diethylaminophenyl)penta-2,4-dienal, 5-(4-methoxyphenyl)penta-2,4-dienal, 5-(3,4-dimethoxyphenyl)penta-2,4-dienal, 5-(2,4-dimethoxyphenyl)penta-2,4-dienal, 5-(4-piperidinophenyl)penta-2,4-dienal, 5-(4-morpholinophenyl)penta-2,4-dienal, 5-(4-pyrrolidinophenyl)penta-2,4-dienal, 5-(4-dimethylamino-1-naphthyl)penta-3,5-dienal, piperonal, 6-nitropiperonal, 2-nitropiperonal, 5-nitrovanillin, 2,5-dinitrosalicylaldehyde, 5-bromo-3-nitrosalicylaldehyde, 3-nitro-4-formylbenzenesulfonic acid, 4-formyl-1-methylpyridinium-, 2-formyl-1-methylpyridinium-, 4-formyl-1-ethylpyridinium-, 2-formyl-1-ethylpyridinium-, 4-formyl-1-benzylpyridinium-, 2-formyl-1-benzylpyridinium-, 4-formyl-1,2-dimethylpyridinium-, 4-formyl-1,3-dimethylpyridinium-, 4-formyl-1-methylquinolinium-, 2-formyl-1-methylquinolinium-, 5-formyl-1-methylquinolinium-, 6-formyl-1-methylquinolinium-, 7-formyl-1-methylquinolinium-, 8-formyl-1-methylquinolinium, 5-formyl-1-ethylquinolinium-, 6-formyl-1-ethylquinolinium-, 7-formyl-1-ethylquinolinium-, 8-formyl-1-ethylquinolinium, 5-formyl-1-benzylquinolinium-, 6-formyl-1-benzylquinolinium-, 7-formyl-1-benzylquinolinium-, 8-formyl-1-benzylquinolinium, 5-formyl-1- allylquinolinium-, 6-formyl-1-allylquinolinium-, 7-formyl-1-allylquinolinium- and 8-formyl-1-allylquinolinium -benzenesulfonate, -p-toluenesulfonate, - methanesulfonate, -perchlorate, -sulfate, -chloride, - bromide, -iodide, -tetrachlorozincate, - methylsulfate, -trifluoromethanesulfonate, -tetrafluoroborate, isatin, 1-methylisatin, 1-allylisatin, 1-hydroxymethylisatin, 5-chloroisatin, 5-methoxyisatin, 5-nitroisatin, 6-nitroisatin, 5-sulfoisatin, 5-carboxyisatin, quinisatin, 1-methylquinisatin and any mixtures of the above compounds.

6. The method according to one of claims 1 to 5, **characterized in that** the bleaching agent B has a pH of from 4 to 11, particularly preferably from 5 to 10, more particularly preferably from 7 to 10.

**Revendications**

1. Procédé de décoloration de fibres de kératine colorées, en particulier de cheveux humains, **caractérisé en ce que**

- un décolorant contenant dans un support cosmétique du peroxyde d'hydrogène est appliquée sur des fibres de kératine colorées avec un colorant, contenant dans un support cosmétique une combinaison d'au moins un composé CH-acide qui est choisi parmi au moins un composé de la formule (CH-1) et/ou d'au moins un composé de la formule (CH-2)

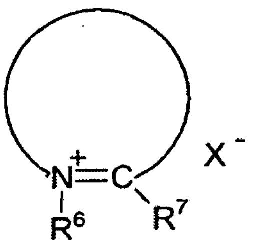
(CH-1)

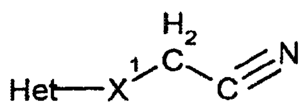
(CH-2)

dans laquelle

• $R^6$ représente un groupe alkyle en $C_1$ à $C_6$ linéaire ou cyclique, un groupe alcényle en $C_2$ à $C_6$, un groupe aryle éventuellement substitué, un groupe hétéroaryle éventuellement substitué, un aryl-alkyle en $C_1$ à $C_6$, un groupe hydroxyalkyle en $C_1$ à $C_6$, un groupe polyhydroxyalkyle en $C_2$ à $C_6$, un groupe alcolxy en $C_1$ à $C_6$-alkyle en $C_1$ à $C_6$, un groupe $R^IR^{II}N-(CH_2)_m-$, dans lequel $R^I$ et R" représentent indépendamment un atome d'hydrogène, un alkyle en $C_1$ à $C_4$, un groupe hydroxyalkyle en $C_1$ à $C_4$ ou un arylalkyle en $C_1$ à $C_6$, $R^I$ et R" pouvant former conjointement avec l'atome d'azote un cycle à 5, 6 ou 7 membres, et m représente le nombre 2, 3, 4, 5 ou 6,

- $R^7$ représente un groupe) alkyle en $C_1$ à $C_6$, en particulier un groupe méthyle,
- $X^-$ représente un anion physiologiquement acceptable,
- le cycle de la formule (CH-1) représente toutes les structures cycliques qui peuvent contenir d'autres hétéroatomes tels que l'azote, l'oxygène ou le soufre et qui peuvent en outre porter des structures cycliques condensées, toutes ces structures cycliques pouvant porter des substituants supplémentaires,
- Het est un groupe hétéro-aromatique éventuellement substitué,
- $X^1$ représente une liaison directe ou un groupe carbonyle,

avec au moins un composé carbonyle réactif, est laissé 1 à 30 minutes sur les fibres puis éliminé par rinçage, et **en ce que**
- l'agent décolorant est exempt de composés peroxo organiques et de persels minéraux.

2. Procédé selon la revendication 1, **caractérisé en ce que** l'agent décolorant contient du peroxyde d'hydrogène dans des quantités comprises entre 0,5 et 12% en poids, de préférence dans des quantités comprises entre 0,5 à 6% en poids, de façon particulièrement préférée dans des quantités comprises entre 0,5 et 4% en poids, de façon tout particulièrement préférée dans des quantités de 0,5 à 3% en poids, calculées à chaque fois sur le poids de tout l'agent.

3. Procédé selon la revendication 1 ou 2, **caractérisé en ce que** l'agent décolorant est laissé 5 à 20 minutes sur les fibres.

4. Procédé selon l'une des revendications 1 à 3, **caractérisé en ce que** le composé CH-acide est choisi parmi au moins un composé du groupe comportant le 2-(2-furoyl)- acétonitrile, le 2-(5-bromo-2-furoyl)-acétonitrile, le 3-(2, 5-diméthyl-3-furyl)-3-oxopropanitril, le 2-(2-thénoyl)-acétonitrile, le 2-(3-thénoyl)-acétonitrile, le 2-(5-fluoro-2-thénoyle)-acétonitrile, le 2-(5-chloro-2-thénoyl)-acétonitrile, le 2-(5-bromo-2-thénoyle)-acétonitrile, le 2-(5-méthyl-2-thénoyl)-acétonitrile, le 2-(2,5-diméthylpyrrol-3-oyl)-acétonitrile, le 2-(1,2,5-triméthylpyrrol-3-oyl)-acétonitrile, le 1*H*-benzimidazol-2-ylacétonitrile, le 1*H*-benzothiazol-2-ylacétonitrile, le 2-(pyrid-2-yl)-àcétonitril, la 2,6-bis(cyanométhyl)pyridine, la 2-(indol-3-oyl)-acétonitrile, le 2-(2-méthyl-indol-3-oyl)-acétonitrile, le 2-(6-hydroxy-4,7-diméthoxy-1-benzofuran-5-oyl)-acétonitrile, et les sels avec un contre-ion physiologiquement acceptable $X^-$ du 1,2-dihydro-1,3,4,6-tétraméthyl-2-oxo-pyrimidinium, du 1, 2-dihydro-1,3-diéthyl-4,6-diméthyl-2-oxo-pyrimidinium, du 1,2-dihydro-1,3-dipropyl-4,6-diméthyl-2-oxo-pyrimidinium, du 1,2-dihydro-1,3-di(2-hydroxyéthyl)-4,6-diméthyl-2-oxo-pyrimidinium, du 1,2-dihydro-1,3-diphényl-4,6-diméthyl-2-oxo-pyrimidinium, du 1,2-dihydro-1,3,4-triméthyl-2-oxo-pyrimidinium, du 1,2-dihydro-1,3-diéthyl-4-méthyl-2-oxo- pyrimidinium, du 1,2-dihydro-1,3-dipropyl-4-méthyl-2-oxo-pyrimidinium, du 1,2-dihydro-1,3-di(2-hydroxyéthyl)-4-méthyl-2-oxo-pyrimidinium, du 1,2-dihydro-1,3-diphényl-4-méthyl-2-oxo-pyrimidinium, 1-allyl-1,2-dihydro-3,4,6-triméthyl-2-oxo-pyrimidinium, du 1,2-dihydro-1-(2-hydroxyéthyl)-3,4,6-triméthyl-2-oxo-pyrimidinium, du 1,2-dihydro-1,3,4,6-tétra-méthyl-2-thioxo-pyrimidinium, du 1,2-dihydro-1,3-diéthyl-4,6-diméthyl-2-thioxo-pyrimidinium, du 1,2-dihydro-1,3-dipropyl-4,6-diméthyl-2-thioxo-pyrimidinium, du 1,2-dihydro-1,3-di(2-hydroxyéthyl)-4,6-diméthyl-2-thioxo-pyrimidinium, du 1,2-dihydro-1,3-diphényl-4,6-diméthyl-2-thioxo-pyrimidinium, du 1,2-dihydro-1,3,4-triméthyl-2-thioxo-pyrimidinium, du 1,2-dihydro-1,3-diéthyl-4-méthyl-2-thioxo-pyrimidinium, du 1,2-dihydro-1,3-dipropyl-4-méthyl-2-thioxo-pyrimidinium, du 1,2-dihydro-1,3-di(2-hydroxyéthyl)-4-méthyl-2-thioxo-pyrimidinium, du 1,2-dihydro-1,3-diphényl-4-méthyl-2-thioxo-pyrimidinium, du 1,2-dihydro-3,4-diméthyl-2-oxo-chinazolinium et du 1,2-dihydro-3,4-diméthyl-2-thioxo-chinazolinium.

5. Procédé selon l'une des revendications 1 à 4, **caractérisé en ce que** le composé carbonyle réactif est choisi parmi au moins un composé du groupe comportnt le benzaldéhyde et ses dérivés, le naphtaldéhyde et ses dérivés, l'aldéhyde cinnamique et ses dérivés, la 2-formylméthylène-1,3,3-triméthylindoline (aldéhyde de Fischer ou aldéhyde tribase), le 2-indolaldéhyde, le 3-indolaldéhyde, le 1-méthylindole-3-aldéhyde, le 2-méthylindole-3-aldéhyde, le 2-(1',3',3'-triméthyl-2-indolinylidène)-acétaldéhyde, le 1-méthylpyrrol-2-aldéhyde, le pyridoxal, le 2,3-dihydro-1,5-diméthyl-3-oxo-2-phényl-1H-pyrazol-4-carboxaldéhyde, le furfural, le 5-nitrofurfural, le chromon-3-aldéhyde, la 3-(5'-nitro-2'-furyl)-acroléin, la 3-(2'-furyl)-acroléine et l'imidazole-2-carbaldéhyde, le 5-(4-diméthylaminophényl)penta-2,4-diénal, le 5-(4-diéthylaminophényl)penta-2,4-diénal, le 5-(4-méthoxy-phényl)penta-2,4- diénal, le 5-(3,4-diméthoxyphényl)penta-2,4-diénal, le 5-(2,4-diméthoxyphényl)penta-2,4-diénal, le 5-(4-pipéridinophényl)penta-2,4-diénal, le 5-(4-morpholinophényl)penta-2,4-diénal, le 5-(4-pyrrolidinophényl)penta-2,4-diénal, le 5-(4-diméthylamino-1-naphtyl)penta-3,5-diénal, le pipéronal, le 6-nitropipéronal, le 2-nitropipéronal, la 5-nitrovanilline, le 2,5-dinitrosalicylaldéhyde, le 5-bromo-3-nitrosalicylaldéhyde, l'acide 3-nitro-4-formylbenzène-sulfonique, les benzène-sulfonate, p-toluène-sulfonate, méthane-sulfonate, perchlorate, sulfate, chlorure, bromure, iodure, tétrachlorozincate, méthylsulfate, trifluorométhane-sulfonate, tétrafluoroborate des 4-formyl-1-méthylpyridinium, 2-formyl-1-méthylpyridinium, 4-formyl-1-éthylpyridinium, 2-formyl-1-éthylpyridinium, 4-formyl-1- benzylpyridinium, 2-formyl-1-benzylpyridinium, 4-formyl-1,2-diméthylpyridinium, 4-formyl-1,3-diméthylpyridinium, 4-formyl-1-méthylquinoléinium, 2-formyl-1-méthylquinoléinium, 5-formyl-1- méthylquinoléinium, 6-formyl-1-méthylquinoléinium, 7-formyl-1-méthylquinoléinium, 8-formyl-1-méthylquinoléinium, 5-formyl-1-éthylquinoléinium, 6-formyl-1-éthylquinoléinium, 7-formyl-1-éthylquinoléinium, 8-formyl-1-éthylquinoléinium, 5-formyl-1-benzylquinoléinium, 6-formyl-1-benzylquinoléinium, 7-formyl-1-benzylquinoléinium, 8-formyl-1-benzylquinoléinium , 5-formyl-1-allylquinoléinium, 6-formyl-1-allylquinoléinium, 7-formyl-1-allylquinoléinium et 8-formyl-1-allylquinoléinium, l'isatine, la 1-méthyl-isatine, la 1-allylisatine, la 1-hydroxyméthyl-isatine, la 5-chloro-isatine, la 5-méthoxy-isatine, la 5-nitroisatine, la 6-nitro-isatine, la 5-sulfo-isatine, la 5-carboxy-isatine, la chinisatine, la 1-méthylchinisatine, et des mélanges quelconques des composés précédents.

6. Procédé selon l'une des revendications 1 à 5, **caractérisé en ce que** l'agent décolorant B a une valeur de pH de 4 à 11, de façon particulière préférée de 5 à 10, de façon tout particulièrement préférée de 7 à 10.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2004022016 A1
- WO 2005120445 A2
- DE 10022743 A1
- DE 3725030 A
- DE 3723354 A
- DE 3926344 A
- DE 4440625 A1
- DE 19503465 A1
- GB 2104091 A
- EP 47714 A
- EP 217274 A
- EP 283817 A
- DE 2817369

### In der Beschreibung aufgeführte Nicht-Patentliteratur

- Deutsche Normen. Benth Verlag GmbH, 1975